# EUROPEAN PATENT APPLICATION

(11) **EP 4 527 296 A1**
(43) Date of publication of application: **26.03.2025**
(21) Application number: 23807624.4
(22) Date of filing: 16.05.2023
(51) Int. Cl.: A61B 6/00, A61B 6/03, G06T 7/00, G06V 10/70

(54) **PROGRAM, INFORMATION PROCESSING METHOD, AND INFORMATION PROCESSING DEVICE**

(30) Priority: 20.05.2022 JP 2022083271
(71) Applicant: Osaka University, Suita-shi, Osaka 565-0871 (JP); Nara Institute Of Science And Technology, Ikoma-shi, Nara 630-0192 (JP)
(72) Inventor: UEMURA, Keisuke, Suita-shi, Osaka 565-0871 (JP); SUGANO, Nobuhiko, Suita-shi, Osaka 565-0871 (JP); TAKAO, Masaki, Suita-shi, Osaka 565-0871 (JP); HAMADA, Hidetoshi, Suita-shi, Osaka 565-0871 (JP); SATO, Yoshinobu, Ikoma-shi, Nara 630-0192 (JP); OTAKE, Yoshito, Ikoma-shi, Nara 630-0192 (JP); GU, Yi, Ikoma-shi, Nara 630-0192 (JP); SOUFI, Mazen, Ikoma-shi, Nara 630-0192 (JP)
(74) Representative: Cabinet Beaumont
(86) International application number: PCT/JP2023/018208
(87) International publication number: WO 2023/224022

(57) **Abstract**

Provided is a program, etc. capable of acquiring information related to an amount of body tissue from an X-ray image with high accuracy using a small number of cases. A computer acquires training data including an X-ray image of a target site and information related to an amount of body tissue obtained from a CT (Computed Tomography) image of the target site. The computer generates a learning model configured to output information related to an amount of body tissue of a target site in an X-ray image when the X-ray image is input using acquired training data.

## Description

### [Technical Field]

The present disclosure relates to a program, an information processing method, and an information processing apparatus.

### [Background Art]

Fractures in the elderly can lead to a decline in daily life functions and may lead to a state of need for nursing care. Therefore, it is considered important to prevent fractures by measuring bone density, diagnosing osteoporosis, and providing appropriate treatment early. For measuring bone density, it is recommended to use a dual-energy X-ray absorptiometry (DXA) device, which measures bone density (bone mass) based on a difference in transmittance of X-rays at two energy levels. However, since the DXA device is a bed-type device that captures an image of a subject in a supine position, an installation space needs to be ensured, and the use is limited despite the high price, so that a distribution rate is low.

Meanwhile, X-ray devices (X-ray devices) are installed in many medical institutions. Non-Patent Documents 1 to 3 propose technology for generating a model that predicts bone density from an X-ray image by training using a pair of the X-ray image and a measurement result (bone density) by a DXA device as training data.

### [Prior Art Document]

### [Non-Patent Document]

[Non-Patent Document 1] Chen-I Hsieh et al., "Automated bone mineral density prediction and fracture risk assessment using plain radiographs via deep learning", NATURE COMMUNICATIONS, 12:5472(2021)
[Non-Patent Document 2] Ryoungwoo Jang et al., "Prediction of osteoporosis from simple hip radiography using deep learning algorithm", Scientific Reports, 11:19997(2021)
[Non-Patent Document 3] Norio Yamamoto et al., "Deep Learning for Osteoporosis Classification Using Hip Radiographs and Patient Clinical Covariates", Biomolecules 2020, 10, 1534

### [Summary of Invention]

### [Problems to be Solved by Invention]

However, the technology disclosed in Non-Patent Documents 1 to 3 requires training using a large amount of training data, and processing load of a collection process and a training process for the training data is large.

An object of the present disclosure is to provide a program, etc. capable of acquiring information related to an amount of body tissue from an X-ray image with high accuracy using a small number of cases.

### [Means for Solving Problems]

A program according to one aspect of the present disclosure causes a computer to execute processes of acquiring training data including an X-ray image of a target site and information related to an amount of body tissue obtained from a CT (Computed Tomography) image of the target site, and generating a learning model configured to output information related to an amount of body tissue of a target site in an X-ray image when the X-ray image is input using acquired training data.

### [Effects of Invention]

According to one aspect of the present disclosure, it is possible to acquire information related to an amount of body tissue from an X-ray image with high accuracy using a small number of cases.

### [Brief Description of Drawings]

FIG. 1 is a block diagram illustrating a configuration example of an information processing apparatus.
FIG. 2 is an explanatory view illustrating an overview of a learning model.
FIG. 3A is an explanatory view illustrating an overview of a learning model.
FIG. 3B is an explanatory view illustrating an overview of a learning model.
FIG. 4 is a flowchart illustrating an example of a generation process procedure for training data.
FIG. 5 is an explanatory view of a process of generating a training DRR image.
FIG. 6 is a flowchart illustrating an example of a generation process procedure for a learning model.
FIG. 7 is a flowchart illustrating an example of an estimation process procedure for bone density.
FIG. 8 is an explanatory view illustrating a screen example.
FIG. 9A is a chart illustrating a relationship between bone density estimated from a predicted DRR image and bone density measured by a DXA device.
FIG. 9B is a chart illustrating a relationship between bone density estimated from a predicted DRR image and bone density measured by a DXA device.
FIG. 10 is an explanatory view illustrating an overview of a learning model of Embodiment 2.
FIG. 11 is a flowchart illustrating an example of a generation process procedure for training data of Embodiment 2.
FIG. 12 is a flowchart illustrating an example of an estimation process procedure for bone density of Embodiment 2.
FIG. 13 is an explanatory view illustrating a screen example.
FIG. 14 is an explanatory view illustrating an overview of a learning model of Embodiment 3.
FIG. 15 is a flowchart illustrating an example of a generation process procedure for training data of Embodiment 3.
FIG. 16 is a flowchart illustrating an example of an estimation process procedure for muscle mass.
FIG. 17 is an explanatory view illustrating a screen example.
FIG. 18 is a flowchart illustrating an example of an estimation process procedure for bone density of Embodiment 4.
FIG. 19 is an explanatory view illustrating a screen example.
FIG. 20 is a flowchart illustrating an example of an estimation process procedure for bone density and muscle mass of Embodiment 5.
FIG. 21 is an explanatory view illustrating a screen example.
FIG. 22 is a flowchart illustrating an example of an alignment process procedure.
FIG. 23A is an explanatory view of an alignment process.
FIG. 23B is an explanatory view of the alignment process.
FIG. 24A is an explanatory view of the alignment process.
FIG. 24B is an explanatory view of the alignment process.
FIG. 25 is a flowchart illustrating an example of a generation process procedure for training data of Embodiment 7.
FIG. 26A is an explanatory view illustrating an effect of an alignment process based on a bone region.
FIG. 26B is an explanatory view illustrating an effect of the alignment process based on the bone region.
FIG. 27 is an explanatory view illustrating a configuration example of a learning model of Embodiment 8.
FIG. 28A is an explanatory view illustrating an effect of a feedback process for loss related to muscle mass.
FIG. 28B is an explanatory view illustrating an effect of a feedback process for loss related to muscle mass.

### [Mode for Carrying out Invention]

Hereinafter, a program, an information processing method, and an information processing apparatus according to the present disclosure will be described with reference to the drawings illustrating embodiments thereof.

### (Embodiment 1)

A description will be given of an information processing apparatus that estimates bone density (information on the amount of body tissue) of a target site based on an X-ray image obtained by capturing the target site by an X-ray device. Bone density of a lumbar vertebra or a proximal femur is generally used to diagnose osteopenia and osteoporosis. Therefore, in this embodiment, a description is given of a configuration in which the target site is the proximal femur, and the bone density of the proximal femur is estimated from an X-ray image (frontal hip joint X-ray image) including the proximal femur within an imaging range. However, the target site is not limited to the proximal femur, and may be other sites such as the lumbar vertebra or thoracic vertebra.

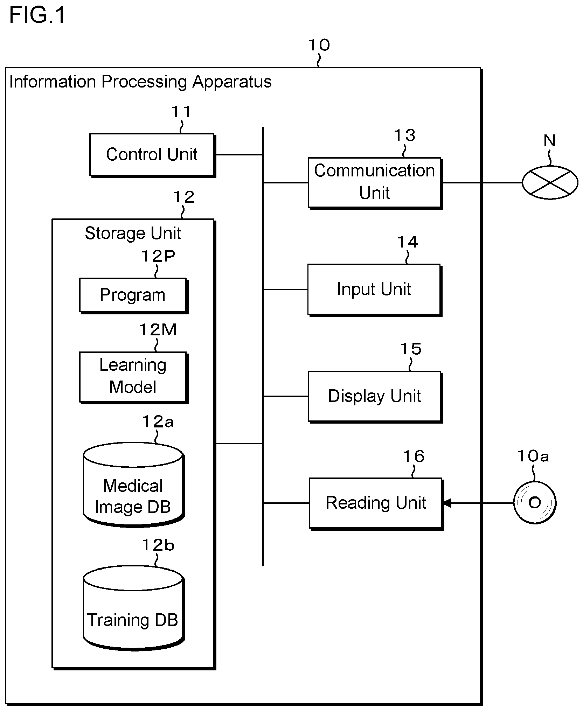

FIG. 1 is a block diagram illustrating a configuration example of an information processing apparatus. The information processing apparatus 10 is an apparatus capable of processing various types of information and transmitting and receiving information, and is, for example, a personal computer, a server computer, a workstation, etc. The information processing apparatus 10 is installed and used in medical institutions, testing institutions, research institutions, etc. The information processing apparatus 10 may be a multi-computer including a plurality of computers, or may be realized by a virtual machine virtually constructed in a single apparatus. When the information processing apparatus 10 is configured as a server computer, the information processing apparatus 10 may be a local server installed in a medical institution, etc., or may be a cloud server connected for communication via a network such as the Internet. In the following description, the information processing apparatus 10 will be described as being one computer.

The information processing apparatus 10 estimates the bone density of the proximal femur based on, for example, the frontal hip joint X-ray image. Specifically, as described later, the information processing apparatus 10 performs machine learning in advance to learn predetermined training data, and prepares a learning model 12M that receives the frontal hip joint X-ray image as input and outputs information on the bone density of the proximal femur in the frontal hip joint X-ray image (information on the amount of body tissue). Then, the information processing apparatus 10 inputs the frontal hip joint X-ray image to the learning model 12M, thereby acquiring information on the bone density of the proximal femur from the learning model 12M. In this embodiment, a DRR image (Digital Reconstructed Radiograph: an X-ray image obtained by projection simulation from a three-dimensional region (region of interest) of a specific site of a CT image) which is a projection image of a three-dimensional region of the proximal femur in a CT image is used as the information on the bone density. Since each pixel value of the CT image is a CT value corresponding to the bone density, a DRR image generated from a specific bone region in the CT image can show a distribution of the bone density, and for example, as the bone density increases, a luminance value (pixel value) increases. Therefore, the learning model 12M of this embodiment is configured to predict and output a DRR image (image representing the amount of body tissue) of the proximal femur contained in the X-ray image when a frontal hip joint X-ray image is input. In addition, the information processing apparatus 10 can acquire the bone density from an X-ray image by calculating the bone density of the proximal femur from the DRR image predicted using the learning model 12M. Since an imaging state in the X-ray image changes when the density of the bone decreases, in this embodiment, the learning model 12M can be used to predict a DRR image corresponding to a state of bone density of the bone region in the X-ray image.

The information processing apparatus 10 has a control unit 11, a storage unit 12, a communication unit 13, an input unit 14, a display unit 15, a reading unit 16, etc., and these respective units are connected to each other via a bus. The control unit 11 includes one or more processors such as a CPU (Central Processing Unit), an MPU (Micro-Processing Unit), a GPU (Graphics Processing Unit), an AI chip (AI semiconductor), etc. The control unit 11 executes a program 12P stored in the storage unit 12 as appropriate, thereby carrying out processing to be performed by the information processing apparatus 10.

The storage unit 12 includes a RAM (Random Access Memory), a flash memory, a hard disk, an SSD (Solid State Drive), etc. The storage unit 12 prestores the program 12P (program product) executed by the control unit 11, various data required for executing the program 12P, etc. In addition, the storage unit 12 temporarily stores data generated when the control unit 11 executes the program 12P. The storage unit 12 further stores the learning model 12M, which will be described later. The learning model 12M is a model trained to output a DRR image of the proximal femur included in a frontal hip joint X-ray image when the X-ray image is input. The learning model 12M is expected to be used as a program module included in artificial intelligence software. The storage unit 12 stores information defining the learning model 12M, such as information on layers included in the learning model 12M, information on nodes included in each layer, and a weight (coupling coefficient) between nodes.

In addition, the storage unit 12 stores a medical image DB 12a and a training DB 12b. The medical image DB 12a stores a frontal hip joint X-ray image and a CT image prepared to train the learning model 12M in association with each other. The medical image used for training includes a frontal hip joint X-ray image and a CT image of a subject diagnosed by a DXA device as having any one of normal bone density, osteoporosis, and osteopenia. The training DB 12b stores training data used in a process of training the learning model 12M, and the training data is stored in the training DB 12b by the information processing apparatus 10 performing a process of generating the training data described below. The learning model 12M, the medical image DB 12a, and the training DB 12b may be stored in another storage device connected to the information processing apparatus 10, or in another storage device with which the information processing apparatus 10 can communicate.

The communication unit 13 is a communication module for connecting to a network N such as the Internet or a LAN (Local Area Network) by wired communication or wireless communication, and exchanges information with other devices via the network N. The input unit 14 receives operation input by a user and transmits a control signal corresponding to operation content to the control unit 11. The display unit 15 is a liquid crystal display or an organic EL display, etc., and displays various information according to an instruction from the control unit 11. A part of the input unit 14 and the display unit 15 may be a touch panel configured as an integrated unit. Note that the input unit 14 and the display unit 15 are not essential, and the information processing apparatus 10 may be configured to receive operation through a connected computer and output information to be displayed to an external display device.

The reading unit 16 reads information stored in a portable storage medium 10a, such as a CD (Compact Disc), a DVD (Digital Versatile Disc), a USB (Universal Serial Bus) memory, an SD card, a micro SD card, a Compact Flash^{®}, etc. The program 12P (program product) and various data stored in the storage unit 12 may be read by the control unit 11 from the portable storage medium 10a via the reading unit 16 and stored in the storage unit 12, or may be downloaded by the control unit 11 from another device via the communication unit 13 and stored in the storage unit 12.

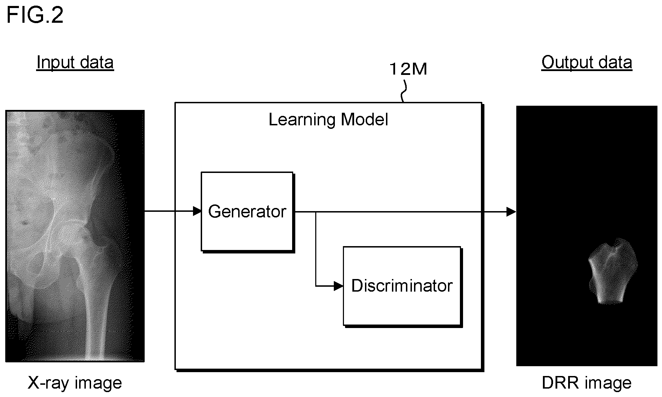

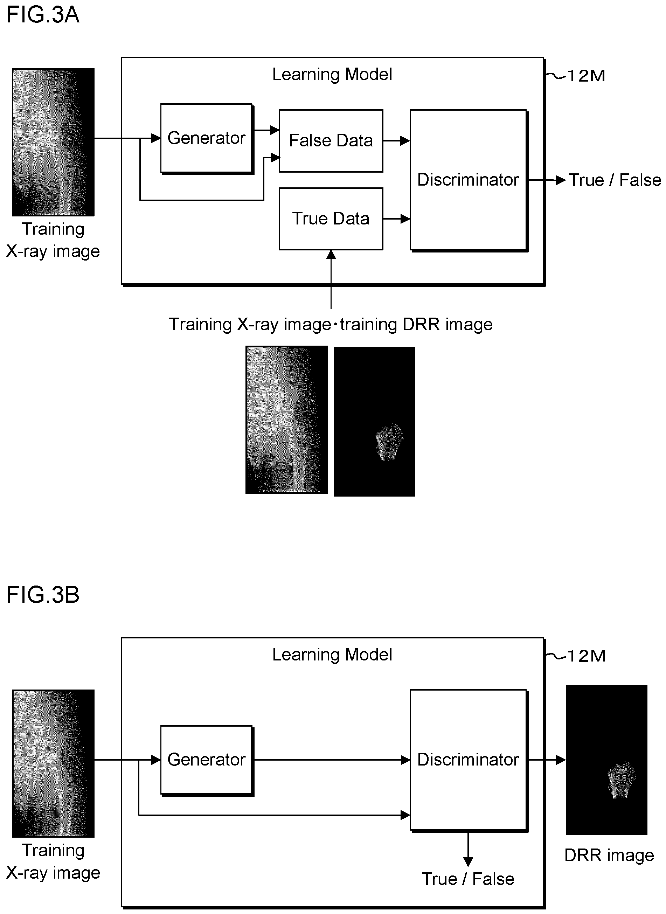

FIG. 2 to FIG. 3B are explanatory views illustrating overviews of the learning model 12M. Note that FIG. 2 conceptually illustrates a state in which a DRR image of a region of interest of the proximal femur is predicted from a half-section image of a frontal hip joint X-ray image, and FIG. 3A and FIG. 3B each conceptually illustrates a state at the time of training the learning model 12M. In addition, FIG. 3A illustrates a state at the time of training a discriminator, and FIG. 3B illustrates a state at the time of training a generator. As illustrated in FIG. 2, the learning model 12M is a model trained to predict a DRR image of the region of interest using a half-section image including the proximal femur on the side targeted for bone density estimation in the frontal hip joint X-ray image as input. Note that a half-section X-ray image input to the learning model 12M may be an X-ray image including a left proximal femur, or may be an X-ray image including a right proximal femur. When the X-ray image including the right proximal femur is input to the learning model 12M, the half-section X-ray image is input to the learning model 12M after being reversed right and left. In this way, it is possible to measure not only the left proximal femur but also the right proximal femur.

In this embodiment, a GAN (Generative Adversarial Network) is used as the learning model 12M. The learning model 12M illustrated in FIG. 2 to FIG. 3B is configured as pix2pix. The GAN includes a generator that generates output data from input data, and a discriminator that identifies authenticity of the data generated by the generator, and the generator and the discriminator compete with each other and are trained in an adversarial manner, thereby constructing a network. The generator is a module having an encoder that extracts latent variables from input data, and a decoder that generates output data from the extracted latent variables.

The learning model 12M is generated by preparing training data that associates a training X-ray image (a half-section image of a frontal hip joint X-ray image) with a training DRR image, and training an untrained learning model using this training data. The training X-ray image and DRR image are preferably a frontal hip joint X-ray image and a DRR image of the subject diagnosed as any one of normal bone density, osteoporosis, and osteopenia by the DXA device. The information processing apparatus 10 of this embodiment generates the learning model 12M trained using the X-ray image and the DRR image prepared for training to predict the DRR image from the X-ray image.

In a training process, the information processing apparatus 10 alternately updates a parameter (weight, etc.) of the generator illustrated in FIG. 3B and a parameter of the discriminator illustrated in FIG. 3A, and ends training when a change in an error function converges. In updating the parameter of the discriminator, the information processing apparatus 10 fixes the parameter of the generator then and inputs the training X-ray image to the generator. The generator receives input of the training X-ray image and generates a DRR image (information on the amount of body tissue at the target site) as output data. Then, the information processing apparatus 10 provides a pair of the X-ray image (training X-ray image) and the DRR image (DRR image generated by the generator) corresponding to input and output of the generator as false data, provides a pair of the X-ray image and the DRR image included in the training data as true data to the discriminator, and causes the discriminator to identify authenticity. The information processing apparatus 10 updates a parameter of the discriminator so that the discriminator outputs a false value when false data is input and outputs a true value when true data is input. The updated parameter is a weight (coupling coefficient), etc. between nodes in the discriminator, and the backpropagation method, the steepest descent method, etc. may be used as a parameter optimization method.

In updating a parameter of the generator, a parameter of the discriminator is fixed and training is performed as illustrated in FIG. 3B. Here, when the training X-ray image is input to the generator and the DRR image generated by the generator is input to the discriminator, the information processing apparatus 10 updates a parameter of the generator so that authenticity is erroneously determined (determined as true), and an image of features similar to those of the training X-ray image (an image gradient is similar, statistics of an output distribution of an intermediate layer of the discriminator are similar, etc.) is generated. Here, the updated parameter is a weight (coupling coefficient), etc. between nodes in the generator, and the backpropagation method, the steepest descent method, etc. may be used as a parameter optimization method. In this way, as illustrated in FIG. 2, the learning model 12M is generated to output a DRR image of a proximal femur in an X-ray image when the X-ray image is input.

The information processing apparatus 10 prepares such a learning model 12M in advance and uses the learning model 12M when generating (predicting) a DRR image from an X-ray image. When actually predicting a DRR image from an X-ray image using the learning model 12M, the information processing apparatus 10 uses only the generator as illustrated in FIG. 2. The learning model 12M may be trained by another learning device. The trained learning model 12M generated by training using another training device is downloaded from the training device to the information processing apparatus 10 via the network N or the portable storage medium 10a, for example, and stored in the storage unit 12. Note that, in the trained learning model 12M, only the generator that generates a DRR image from an X-ray image may be downloaded from the training device to the information processing apparatus 10.

The learning model 12M may be a GAN such as CycleGAN, StarGAN, etc., in addition to pix2pix. In addition, the learning model 12M is not limited to the GAN, and may be a neural network such as a Variational Autoencoder (VAE) or a Convolutional Neural Network (CNN) (for example, U-net), or a model based on another learning algorithm, or may be configured by combining a plurality of learning algorithms.

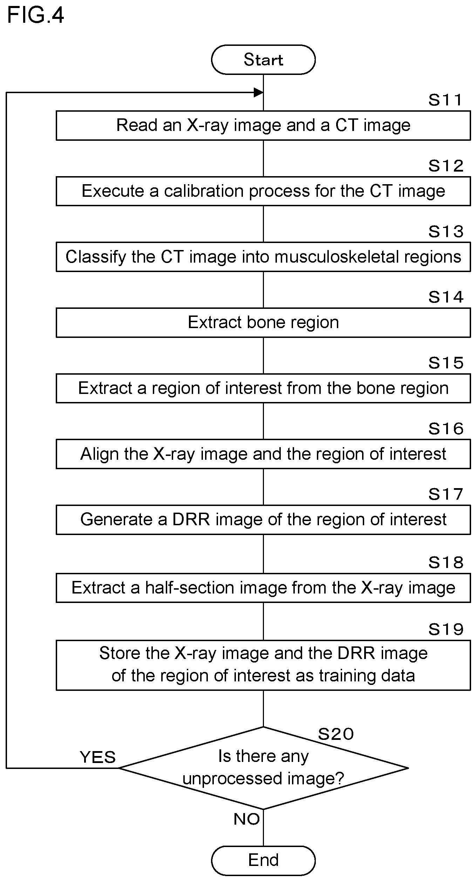

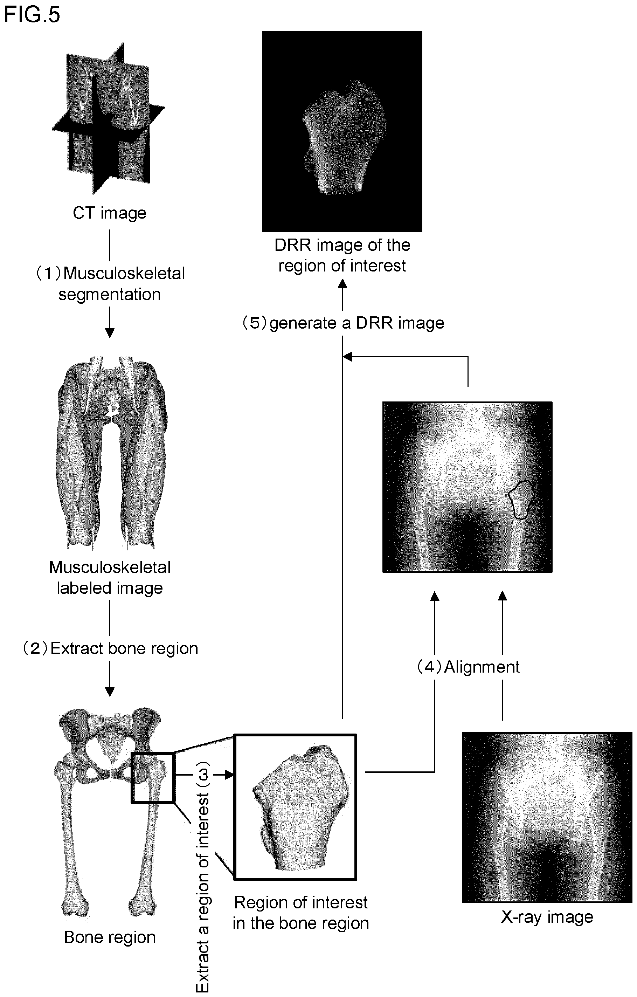

Here, a description will be given of a process of generating a training DRR image used for training the learning model 12M. FIG. 4 is a flowchart illustrating an example of a generation process procedure for training data, and FIG. 5 is an explanatory view of a process of generating a training DRR image. The following process is executed by the control unit 11 of the information processing apparatus 10 in accordance with the program 12P stored in the storage unit 12, but may be executed by another information processing apparatus or training device. In the following process, it is assumed that, as an X-ray image and a CT image used to generate training data, a pair of a frontal hip joint X-ray image and a CT image obtained by capturing a region including a pelvis and right and left femurs of a subject is each associated and stored in the medical image DB 12a.

The control unit 11 of the information processing apparatus 10 reads one pair of a frontal hip joint X-ray image and a CT image stored in the medical image DB 12a (S11). First, the control unit 11 executes a luminance value calibration process for the read CT image (S12), and corrects each luminance value (CT value) in the CT image. The luminance value (CT value) of each pixel measured by CT varies due to individual differences in X-ray CT device, differences in installation environment, capturing condition, etc., and thus calibration is required to correct variation of a measurement value. For example, a calibration process for a CT image is performed using calibration data acquired, for example, when the X-ray CT device is installed, when a part such as a bulb is replaced, when imaging starts, or periodically. Calibration data is generated by capturing a phantom made of a material having known characteristics using the X-ray CT device, based on obtained radiation density (CT value expressed in HU (Hounsfield units)) and tissue density of the material. Specifically, a conversion formula for converting the radiation density obtained by passing through the phantom into the tissue density of the material is used for the calibration data.

In addition, the calibration process can use a method described in an article entitled "Automated segmentation of an intensity calibration phantom in clinical CT images using a convolutional neural network" by the present inventor, Keisuke Uemura et al., published in the International Journal of Computer Assisted Radiology and Surgery (IJCARS) (published online on March 17, 2021). The article discloses technology for capturing a phantom including a substance having a plurality of known tissue densities (a substance having different calcium contents) together with a subject using the X-ray CT device and automatically extracting a captured region of each substance of the phantom from the obtained CT image by using a CNN. By using the technology disclosed in the article, based on a CT value (radiation density) of the captured region of each substance extracted from the CT image, it is possible to generate calibration data for converting the CT value into tissue density of each substance. By using the calibration data generated in this way to perform a calibration process on the CT value of the captured region of the subject, it is possible to acquire accurate tissue density in the subject.

Next, the control unit 11 performs a process of classifying each pixel in the CT image, which has been calibrated in step S12, into one of a plurality of regions (musculoskeletal regions) including a bone region, a muscle region, and other regions, using a segmentation DNN (Deep Neural Network) (S13). The process of classifying each pixel in the CT image into the musculoskeletal regions can be performed, for example, using a method described in an article entitled "Automated Muscle Segmentation from Clinical CT Using Bayesian U-Net for Personalized Musculoskeletal Modeling" by the present inventor, Yoshito Otake, et al., published on pages 1030-1040 of IEEE Transactions on Medical Imaging, VOL. 39, No. 4, April 2020. The article discloses a musculoskeletal segmentation model that receives a CT image as input, classifies each pixel in the input CT image as any one of a bone region, a muscle region, or another region, and outputs a classified CT image (musculoskeletal labeled image) in which each pixel is associated with a label for each region. The musculoskeletal segmentation model disclosed in the article is configured as Bayesian U-net. In this way, as illustrated in (1) of FIG. 5, from a CT image, it is possible to acquire a musculoskeletal labeled image in which each pixel in the CT image is classified into one of three regions and a label is associated with each region. Note that, in FIG. 5, each pixel in the musculoskeletal labeled image is represented diagrammatically by a color (shade) according to the classified region and muscle type, etc.

As illustrated in (2) of FIG. 5, the control unit 11 extracts bone region data from the CT image based on the musculoskeletal labeled image generated from the CT image (S14). Then, as illustrated in (3) of FIG. 5, the control unit 11 extracts a region of interest (here, left proximal femur data) from the extracted bone region data (CT image) (S15). Note that, for example, a process of extracting the region of interest from the bone region can be performed by pattern matching using a template. In this case, a template indicating a shape of the left proximal femur is stored in advance in the storage unit 12, and the control unit 11 determines whether or not there is a region that matches the template from the CT image of the bone region, and extracts the region that matches the template from the bone region, thereby extracting data on the region of interest in the bone region. Note that, for example, a process of extracting the region of interest from the bone region can be performed using a learning model machine-trained to output the region of interest (the region of the left proximal femur) in the bone region when a CT image of the bone region is input. In this case, the control unit 11 inputs the CT image of the bone region to the learning model, and can specify and extract the region of interest in the bone region based on output information from the learning model.

Next, the control unit 11 aligns a capturing target (here, the left proximal femur) in two images in the X-ray image (frontal hip joint X-ray image) acquired in step S11 and the region of interest in the CT image extracted in step S15 (S16). With respect to the X-ray image, the control unit 11 detects a luminance gradient (edge) of the image based on the pixel value of each pixel, and specifies a capturing target in the X-ray image based on the detected luminance gradient. Note that the control unit 11 may specify the capturing target in the X-ray image by pattern matching using a template prepared in advance, or using a learning model trained in advance. Then, with regard to the region of interest (the left proximal femur, which is the capturing target) in the CT image, the control unit 11 specifies a capturing direction that matches the capturing target in the X-ray image and generates a CT image of the region of interest viewed in the specified direction. In this way, as illustrated in (4) of FIG. 5, it is possible to acquire a CT image of the region of interest aligned with the capturing target in the X-ray image.

Note that the capturing target in the X-ray image and the capturing target in the CT image can be aligned using, for example, a method described in an article entitled "Can Anatomic Measurements of Stem Anteversion Angle Be Considered as the Functional Anteversion Angle?" by the present inventor, Keisuke Uemura et al., published on pages 595-600 of The Journal of Arthroplasty 33 (2018). The article discloses technology for specifying the pelvis and the femur in the CT image by performing segmentation using a hierarchical statistical shape model on the CT image of the pelvis and the femur, and aligning (associating) the pelvis and the femur in the CT image and the pelvis and the femur in the X-ray image. In addition, the X-ray image and the CT image can be aligned using a method described in an article entitled "3D-2D registration in mobile radiographs: algorithm development and preliminary clinical evaluation" by the present inventor, Yoshito Otake, et al., published on pages 2075-2090 of Physics in Medicine and Biology 60 (2015). This article discloses technology for generating a CT image of the capturing target viewed in the same direction as the capturing direction of the X-ray image by translating and rotating the CT image.

Then, from a CT image of the region of interest (left proximal femur) aligned with the capturing target (left proximal femur) in the X-ray image, the control unit 11 generates a DRR image of the region of interest by projecting each pixel of the CT image in the same direction as the capturing direction of the X-ray image (S17). The control unit 11 calculates an integrated value of each pixel value (luminance value, voxel value) arranged in the same direction as the capturing direction of the X-ray image in the CT image, and sets the calculated integrated value as each pixel value of the DRR image of the region of interest. In this way, the DRR image of the region of interest illustrated in (5) of FIG. 5 is obtained, and each pixel value in the DRR image corresponds to bone density of each position.

The control unit 11 extracts a half-section image including the left proximal femur from the frontal hip joint X-ray image acquired in step S11 (S18). Specifically, the control unit 11 extracts a right half region (region including the left femur) obtained by dividing the frontal hip joint X-ray image in half at a center in a left-right direction. The control unit 11 associates the extracted X-ray image (half-section image of the frontal hip joint X-ray image) with the DRR image of the region of interest generated in step S17, and stores the images as training data in the training DB 12b (S19). The control unit 11 determines whether or not there is any unprocessed image on which the above-mentioned training data generation process has not been performed among the X-ray image and CT image stored in the medical image DB 12a (S20). When it is determined that there is an unprocessed image (S20: YES), the control unit 11 returns to processing of step S11 and executes processing of steps S11 to S19 on the X-ray image and CT image on which the training data generation process has not been performed. When it is determined that there is no unprocessed image (S20: NO), the control unit 11 ends the series of processes. By the above-mentioned processing, based on the X-ray image and the CT image stored in the medical image DB 12a, training data used to train the learning model 12M can be generated and accumulated in the training DB 12b. In the above-mentioned processing, a description has been given of an example in which the X-ray image and the CT image used to generate the training data are stored in the medical image DB 12a. However, the control unit 11 may be configured to acquire an X-ray image and a CT image stored in another device, for example, via the network N or the portable storage medium 10a. For example, the control unit 11 may be configured to acquire an X-ray image and a CT image from electronic medical record data stored in an electronic medical record server. In addition, in the above-mentioned processing, the region of interest of the bone region is extracted from the CT image and then aligned with the X-ray image. However, the region of interest may be extracted from the CT image after alignment with the X-ray image.

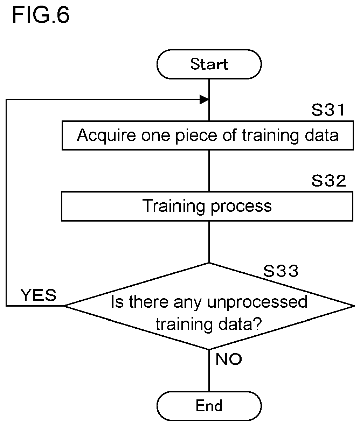

Next, a description will be given of a process of generating the learning model 12M by training using training data generated by the above-mentioned processing. FIG. 6 is a flowchart illustrating an example of a generation process procedure for the learning model 12M. The following process is executed by the control unit 11 of the information processing apparatus 10 in accordance with the program 12P stored in the storage unit 12. However, the following process may be executed by another training device. Furthermore, the process of generating the training data illustrated in FIG. 4 and the process of generating the learning model 12M illustrated in FIG. 6 may be executed by different devices.

The control unit 11 of the information processing apparatus 10 acquires one piece of training data from the training DB 12b (S31). Specifically, the control unit 11 reads one pair of a DRR image and a half-section image (specifically, an X-ray image including the region of the left proximal femur) of a frontal hip joint X-ray image stored in the training DB 12b. The control unit 11 performs a process of training the learning model 12M using the read training data (S32). Here, the control unit 11 updates parameters of the generator and the discriminator of the learning model 12M according to the above-mentioned procedure, and generates the learning model 12M that generates and outputs a DRR image of the proximal femur in an X-ray image included in the training data when the X-ray image is input.

The control unit 11 determines whether or not there is any unprocessed training data on which a training process has not been performed in the training data stored in the training DB 12b (S33). When it is determined that there is unprocessed training data (S33: YES), the control unit 11 returns to processing of step S31 and executes processing of steps S31 to S32 for the training data on which the training process has not been performed. When it is determined that there is no unprocessed training data (S33: NO), the control unit 11 ends a series of processes. The above-mentioned training process generates the learning model 12M that receives input of an X-ray image including a region of a proximal femur, thereby outputting a DRR image of the proximal femur.

By repeatedly performing the training process using the training data as described above, the learning model 12M can be further optimized. In addition, the previously trained learning model 12M can be retrained by performing the above-mentioned training process. In this case, the learning model 12M with higher accuracy can be generated. Note that the learning model 12M of this embodiment is trained using training data that allows for spatial correspondence between abundant 3D data obtained by CT and an X-ray image with high accuracy using segmentation technology that accurately classifies a musculoskeletal region from a CT image and technology that accurately aligns a target site in the X-ray image and a target site in the CT image. For this reason, it is possible to realize the learning model 12M capable of generating a highly accurate DRR image without requiring a large number of cases (training data).

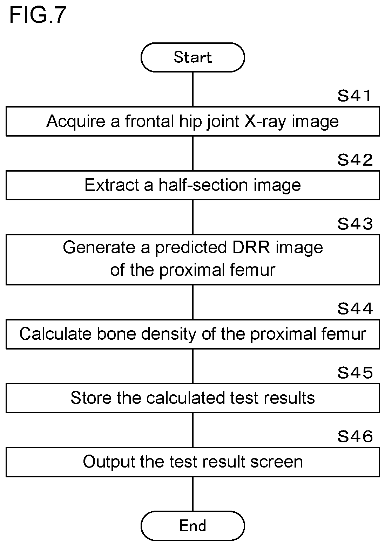

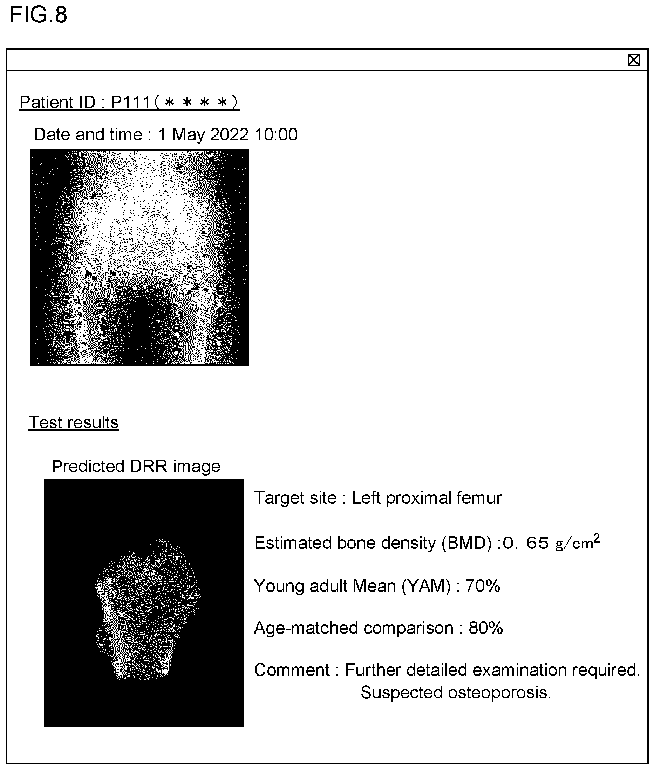

Next, a description will be given of a process of estimating the bone density of the proximal femur from the frontal hip joint X-ray image including the proximal femur of the subject using the learning model 12M generated as described above. FIG. 7 is a flowchart illustrating an example of an estimation process procedure for bone density. FIG. 8 is an explanatory view illustrating a screen example. The following process is executed by the control unit 11 of the information processing apparatus 10 according to the program 12P stored in the storage unit 12. In the following, the DRR image generated from the X-ray image using the learning model 12M is referred to as a predicted DRR image.

The control unit 11 of the information processing apparatus 10 acquires a frontal hip joint X-ray image of a region including the pelvis and the right and left femurs of the subject such as a patient, captured by the X-ray device (S41). For example, the control unit 11 acquires the frontal hip joint X-ray image of the patient for whom bone density is to be estimated from electronic medical record data stored in the electronic medical record server. In addition, when the frontal hip joint X-ray image of the subject is stored in the portable storage medium 10a, the control unit 11 may read the frontal hip joint X-ray image from the portable storage medium 10a using the reading unit 16.

The control unit 11 extracts a half-section image including the proximal femur on the side where bone density is to be measured from the acquired frontal hip joint X-ray image (S42). Specifically, the control unit 11 extracts the right half region (region including the left femur) obtained by dividing the frontal hip joint X-ray image in half at the center in the left-right direction. Note that, when measuring the bone density of the right proximal femur of the subject, the control unit 11 extracts a left half region (including the right femur) of the frontal hip joint X-ray image and then performs a process of reversing the image right and left. Based on the half-section image of the X-ray image extracted in step S42, the control unit 11 generates a predicted DRR image of the proximal femur in the X-ray image (S43). Specifically, the control unit 11 inputs the X-ray image including the proximal femur (the half-section image of the frontal hip joint X-ray image) to the learning model 12M and acquires the predicted DRR image of the proximal femur in the X-ray image as output information from the learning model 12M.

The control unit 11 calculates the bone density of the proximal femur from the generated predicted DRR image of the proximal femur (S44). Each pixel value of the predicted DRR image is a value corresponding to the bone density, and the control unit 11 calculates the bone density of the proximal femur, for example, by calculating an average value of each pixel value in the predicted DRR image. In addition to the bone density (bone mineral density (BMD)), the control unit 11 calculates a young adult comparison result (YAM: Young Adult Mean) and an age-matched comparison result calculated from the bone density. The control unit 11 stores the calculated test results in, for example, the electronic medical record data in the electronic medical record server (S45).

The control unit 11 generates a screen for displaying the test results, outputs the screen to, for example, the display unit 15 (S46), and ends the process by displaying the screen on the display unit 15. For example, the control unit 11 generates a test result screen as illustrated in FIG. 8. The screen illustrated in FIG. 8 displays subject identification information (for example, patient ID, patient name, etc.), the frontal hip joint X-ray image, and an imaging date and time thereof. Furthermore, the screen illustrated in FIG. 8 displays, as the test results for the bone density based on the frontal hip joint X-ray image, a predicted DRR image, a name of a target site in the predicted DRR image (left proximal femur in FIG. 8), bone density estimated from the predicted DRR image, young adult mean, and age-matched comparison. In addition, for example, when comments to be presented to a doctor, etc. are stored in the storage unit 12 in association with each numerical value of bone density, young adult mean, or age-matched comparison, the control unit 11 may read the comments corresponding to the calculated test results (bone density, young adult mean, or age-matched comparison) from the storage unit 12 and display the comments on the test result screen as illustrated in FIG. 8.

By the above-mentioned processing, it is possible to estimate the bone density of the proximal femur in a frontal hip joint X-ray image captured using the X-ray device generally used in a medical institution, etc., from the frontal hip joint X-ray image. In addition, in this embodiment, it is possible to present to the doctor, etc., the predicted DRR image of the proximal femur generated from the frontal hip joint X-ray image and the bone density estimated from the predicted DRR image. Therefore, the doctor can determine a state of the proximal femur of the patient based on the predicted DRR image and the estimated bone density.

In this embodiment, the learning model 12M automatically extracts features of the imaging state of the proximal femur in the X-ray image to generate the predicted DRR image, so that the bone density can be estimated by simply capturing an image using the X-ray device without performing a test using the DXA device, etc. Therefore, it is possible to estimate the bone density of the target site from an X-ray image captured in a health checkup or at a small clinic, so that a bone density measurement test can be easily performed. Therefore, early diagnosis and early treatment intervention of osteopenia or osteoporosis are possible, and it is expected that osteopenia or osteoporosis-related fractures can be prevented and that this contributes to extension of healthy life expectancy.

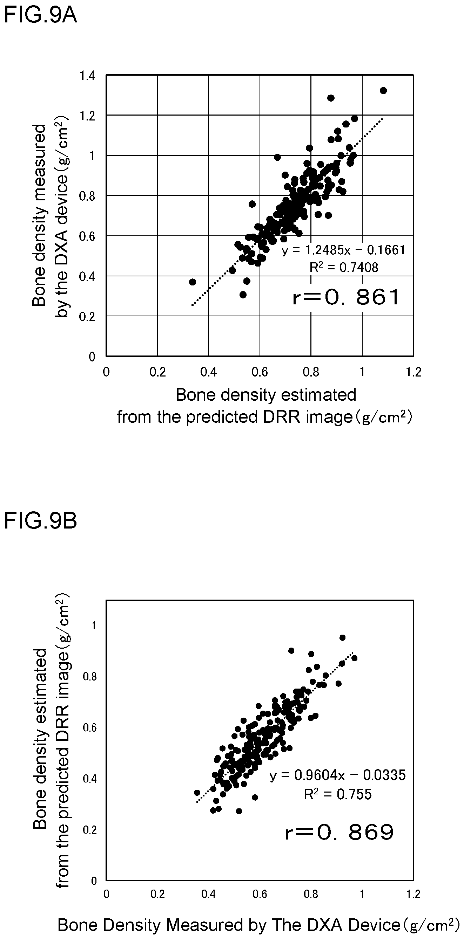

In this embodiment, as described above, it is possible to realize highly accurate spatial alignment between the target site (proximal femur) in the X-ray image and the target site in the CT image. Therefore, by using training data based on the highly accurately aligned X-ray image and CT image (DRR image), it is possible to generate a highly accurate predicted DRR image without learning a large amount of training data. For example, a predicted DRR image is generated from an X-ray image using the learning model 12M trained using 200 pairs of training data generated from the X-ray image and a CT image collected from the patient having osteoarthritis of the hip joint, and a result of comparison between bone density of a proximal femur estimated from the predicted DRR image and bone density of the proximal femur measured using the DXA device is illustrated in FIG. 9A and FIG. 9B. FIG. 9A and FIG. 9B are charts each indicating a relationship between the bone density estimated from the predicted DRR image and the bone density measured using the DXA device. Each of the chart illustrated in FIG. 9A and the chart illustrated in FIG. 9B illustrates a verification result based on X-ray images and bone mineral density (BMD) collected by the X-ray device and the DXA device at a different medical institution.

In the chart of FIG. 9A, a horizontal axis represents the bone density of the proximal femur estimated from the predicted DRR image, and a vertical axis represents the bone density of the proximal femur measured by the DXA device. In the chart of FIG. 9B, a horizontal axis represents the bone density of the proximal femur measured by the DXA device, and a vertical axis represents the bone density of the proximal femur estimated from the predicted DRR image. As can be seen from the charts of FIG. 9A and FIG. 9B, there is a high linear correlation between the bone density of the proximal femur estimated from the predicted DRR image and the bone density of the proximal femur measured by the DXA device. Specifically, at the medical institution illustrated in FIG. 9A, a correlation coefficient of 0.861 was obtained, and an average error (mean absolute error) between the bone density estimated from the predicted DRR image and the bone density measured by the DXA device was 0.06 g/cm2. In addition, at the medical institution illustrated in FIG. 9B, a correlation coefficient of 0.869 was obtained, and an average error between the bone density estimated from the predicted DRR image and the bone density measured by the DXA device was 0.07 g/cm2. In this way, even when the learning model 12M is trained using a small amount of training data, it is possible to predict bone density to the same extent as that of the measurement results using the DXA device. Therefore, workload in a collection process and a training process for the training data can be reduced.

In this embodiment, a description has been given of a configuration in which the predicted DRR image of the proximal femur is generated from the X-ray image including the proximal femur using the learning model 12M, and the bone density of the proximal femur is estimated from the predicted DRR image. However, a site targeted for bone density estimation may be a lumbar vertebra, a thoracic vertebra, a cervical vertebra, a clavicle, a rib, a bone of a hand, a bone of a foot, or specific sites thereof, etc. in addition to the proximal femur. For other sites, the same processing is performed to generate training data and generate a learning model, and it is possible to estimate the bone density using the learning model.

In this embodiment, the process of generating training data, the process of training the learning model 12M using the training data, and the process of estimating bone density using the learning model 12M are not limited to being performed locally by the information processing apparatus 10. For example, an information processing apparatus may be provided to perform each of the above-mentioned processes. In addition, a server may be provided to perform the process of generating training data and the process of training the learning model 12M. In this case, the information processing apparatus 10 is configured to transmit the X-ray image and the CT image used for training data to the server, and the server is configured to generate training data from the X-ray image and the CT image, generate the learning model 12M by the training process using the generated training data, and transmit the learning model 12M to the information processing apparatus 10. Therefore, the information processing apparatus 10 can realize the process of estimating the bone density of the target site using the learning model 12M acquired from the server. In addition, the server may be provided to perform the process of estimating the bone density using the learning model 12M. In this case, the information processing apparatus 10 is configured to transmit an X-ray image of the subject to the server, and the server is configured to perform the process of generating the predicted DRR image using the learning model 12M and the process of estimating the bone density, and transmit the generated predicted DRR image and the estimated bone density to the information processing apparatus 10. Even in such a configuration, the same processing as that in the above-mentioned embodiment is possible, and the same effect can be obtained.

### (Embodiment 2)

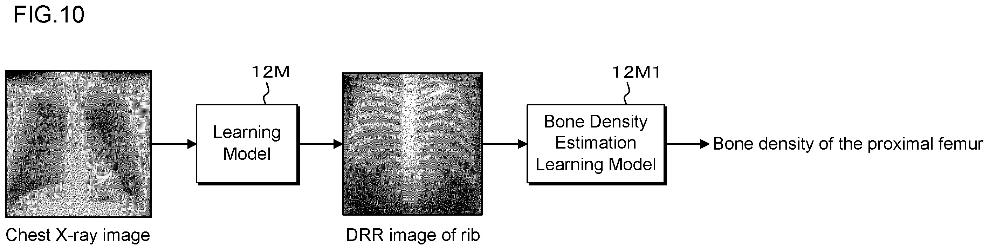

In the above-mentioned Embodiment 1, a description has been given of a configuration in which the predicted DRR image of the target site (for example, the proximal femur) is generated from the X-ray image of the target site, and the bone density of the target site is estimated from the predicted DRR image. In this embodiment, a description is given of an information processing apparatus that estimates bone density of a target site at which bone density is to be estimated from an X-ray image of a site different from the target site at which the bone density is to be estimated. The information processing apparatus of this embodiment has a similar configuration to that of the information processing apparatus 10 of Embodiment 1, and thus a description of the configuration will be omitted. Note that, in addition to the configuration illustrated in FIG. 1, the information processing apparatus 10 of this embodiment stores a bone density estimation learning model 12M1 (see FIG. 10) in the storage unit 12. Furthermore, the medical image DB 12a of this embodiment stores an X-ray image and a CT image of a site different from a target site at which bone density is to be estimated, and bone density measured by the DXA device for the target site at which bone density is to be estimated, in association with each other. In this embodiment, bone density of the proximal femur is estimated from a chest X-ray image, so that the medical image DB 12a stores an X-ray image and a CT image of a chest of the subject and bone density of the proximal femur of the subject measured by the DXA device. Note that the bone density stored in the medical image DB 12a may be bone density of a site used in diagnosis of osteopenia and osteoporosis, and may be the bone density of the lumbar vertebra, the pelvis, or the femur or may be an average value or a median value of bone density of the entire body.

FIG. 10 is an explanatory view illustrating an overview of the learning models 12M and 12M1 of Embodiment 2. When estimating the bone density of the proximal femur, the information processing apparatus 10 of this embodiment generates a predicted DRR image of a site different from the proximal femur, for example, a site such as a rib, a clavicle, or a thoracic vertebra captured by chest X-ray photography, from the X-ray image of the site using the learning model 12M. Then, the information processing apparatus 10 estimates the bone density of the proximal femur from the generated predicted DRR image using the bone density estimation learning model 12M1 (second learning model). Note that a captured site of the X-ray image used to estimate the bone density of the proximal femur is not limited to the rib, clavicle, or thoracic vertebra, and each bone in the X-ray image in which the proximal femur is not captured can be used.

The bone density estimation learning model 12M1 is a machine learning model trained using predetermined training data, and is trained to receive input of a DRR image generated from a CT image of the chest of the subject and output the bone density of the proximal femur of the subject. The bone density estimation learning model 12M1 is expected to be used as a program module included in artificial intelligence software. The bone density estimation learning model 12M1 is configured using, for example, a CNN, but may be configured using another algorithm such as logistic regression or linear regression, or may be configured by combining a plurality of algorithms. Since bone density of human bones is considered to decrease in the same manner in each bone of the body, it is considered that there is a strong correlation between the bone density of the bone in the capturing target in the CT image and the bone density of the bone in a site other than the capturing target. Therefore, in this embodiment, the bone density estimation learning model 12M1 can be used to estimate the bone density in the site other than the capturing target, which is predicted from the CT image (DRR image).

The bone density estimation learning model 12M1 is generated by preparing training data that associates a training DRR image with training (ground truth) bone density, and using this training data to machine-train the untrained learning model 12M1. For example, a DRR image generated from a CT image obtained by capturing the chest of the subject diagnosed as any one of normal bone density, osteoporosis, and osteopenia by the DXA device can be used as the training DRR image, and bone density of the proximal femur of the subject measured using the DXA device can be used as the training bone density. The information processing apparatus 10 of this embodiment performs training using a DRR image and bone density prepared for training, and generates the bone density estimation learning model 12M1 that outputs, from the DRR image, bone density of a site other than the capturing target of the DRR image.

The bone density estimation learning model 12M1 is trained to output bone density of a ground truth when a training DRR image is input. In the training process, the learning model 12M1 performs calculation based on the input DRR image and calculates an output value that is a calculation result. Then, the learning model 12M1 compares the calculated output value with the bone density of the ground truth and optimizes a parameter used in the calculation process so that the two values are close to each other. The parameter is a weight (coupling coefficient), etc. between nodes in the bone density estimation learning model 12M1, and the backpropagation method, the steepest descent method, etc. may be used as a parameter optimization method. In this way, the learning model 12M1 is generated to output the bone density of the proximal femur when a DRR image generated from a chest CT image is input. The bone density estimation learning model 12M1 may be trained by another training device.

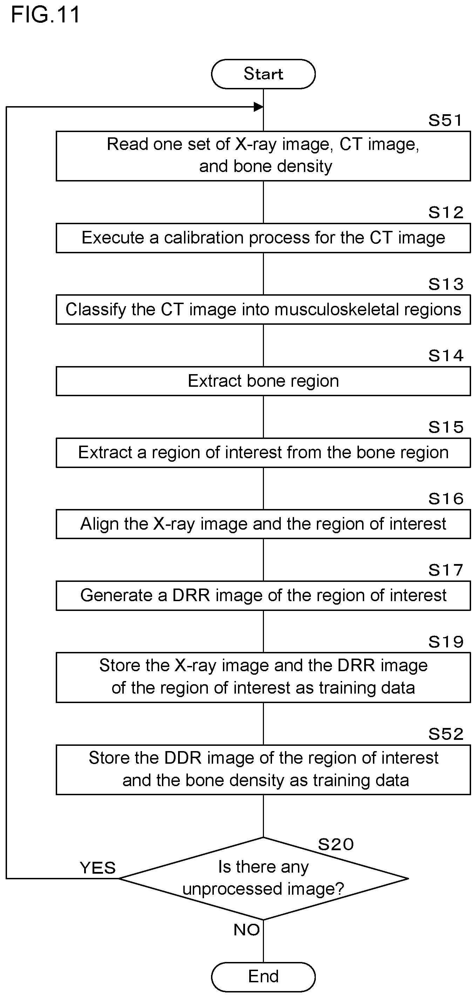

FIG. 11 is a flowchart illustrating an example of a generation process procedure for training data of Embodiment 2. Processing illustrated in FIG. 11 is obtained by adding step S51 instead of step S11, deleting step S18, and adding step S52 between steps S19 and S20 in the processing illustrated in FIG. 4. A description of the same steps as those of FIG. 4 will be omitted. Note that, in this embodiment, it is assumed that a chest X-ray image and a chest CT image obtained by capturing a chest of each subject and bone density of a proximal femur of each subject measured by the DXA device are stored in the medical image DB 12a in association with each other to train the learning models 12M and 12M1.

The control unit 11 of the information processing apparatus 10 of this embodiment reads one set of a chest X-ray image, a chest CT image, and bone density of a proximal femur from the medical image DB 12a (S51). Then, the control unit 11 executes similar processing to that of steps S12 to S17 and S19 of FIG. 4. That is, the control unit 11 executes a calibration process on the chest CT image (S12), classifies each pixel of the chest CT image as a bone region, a muscle region, or another region (S13), and extracts data of the bone region from the chest CT image (S14). Then, the control unit 11 extracts data of a region of interest (for example, a rib) from the CT image of the extracted bone region (S15), and aligns a capturing target (here, a rib) between the chest X-ray image acquired in step S51 and the region of interest in the CT image extracted in step S15 (S16). Furthermore, the control unit 11 generates a DRR image of the region of interest from the CT image of the region of interest (ribs) that has been aligned (S17), and stores the X-ray image acquired in step S51 and the DRR image of the region of interest generated in step S17 in the training DB 12b in association with each other as training data for training the learning model 12M (S19). Note that, in this embodiment, the chest X-ray image read from the medical image DB 12a is used as training data without change. However, as in step S18 of FIG. 4, a process of extracting a region to be used as training data from the read X-ray image may be performed.

Furthermore, the control unit 11 associates the DRR image of the region of interest generated in step S17 with the bone density of the proximal femur acquired in step S51, and stores the DDR image and the bone density in the storage unit 12 as training data for training the bone density estimation learning model 12M1 (S52). Note that the control unit 11 may prepare, in the storage unit 12, a DB for storing training data for learning the bone density estimation learning model 12M1, and store the generated training data in this DB. Thereafter, the control unit 11 proceeds to the process of step S20. Through the above-mentioned processing, based on the X-ray image, the CT image, and the bone density stored in the medical image DB 12a, training data used to train the learning model 12M can be generated and stored in the training DB 12b, and training data used to train the bone density estimation learning model 12M1 can be generated and stored in the DB.

In this embodiment, generation of the bone density estimation learning model 12M1 can be realized by a process similar to the training process illustrated in FIG. 6. Note that, in the process of generating the bone density estimation learning model 12M1, in step S31 of FIG. 6, the control unit 11 reads a pair of a DRR image of a region of interest (here, a rib) and bone density of a proximal femur stored in the storage unit 12. In addition, in step S32, the control unit 11 inputs the DRR image included in the training data to the bone density estimation learning model 12M1 and acquires output information from the bone density estimation learning model 12M1. The control unit 11 compares the acquired output information with the bone density of the proximal femur included in the training data, and optimizes a parameter such as a weight between nodes in the bone density estimation learning model 12M1, for example, by using the backpropagation method, so that the two are close to each other. By the above-mentioned processing, the bone density estimation learning model 12M1 that outputs the bone density of the proximal femur when inputting the DRR image generated from the CT image of the rib is generated.

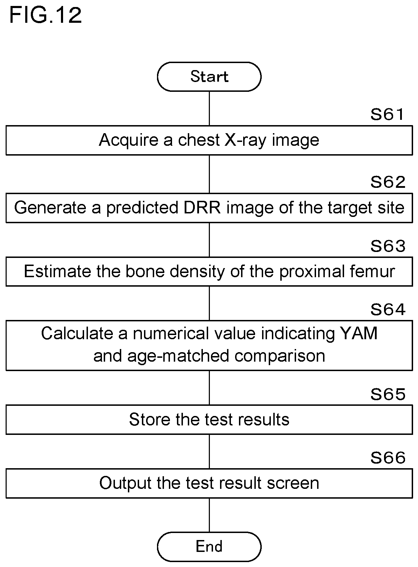

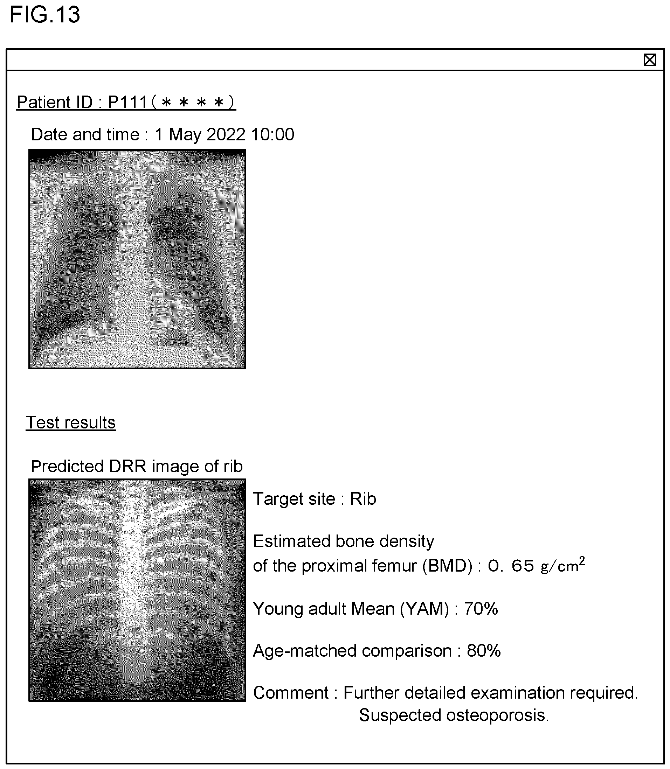

Next, a description will be given of a process of estimating the bone density of the proximal femur of the subject from the chest X-ray image of the subject using the learning models 12M and 12M1. FIG. 12 is a flowchart illustrating an example of an estimation process procedure for bone density of Embodiment 2, and FIG. 13 is an explanatory view illustrating a screen example. The control unit 11 of the information processing apparatus 10 acquires a chest X-ray image obtained by capturing the chest of the subject using the X-ray device (S61). Here, for example, the control unit 11 acquires the chest X-ray image of the subject for which bone density is to be estimated from electronic medical record data.

The control unit 11 inputs the acquired chest X-ray image to the learning model 12M and generates a predicted DRR image of the target site (here, a rib) in the X-ray image (S62). The control unit 11 acquires the predicted DRR image of the rib as output information of the learning model 12M. Note that, in this embodiment, the acquired chest X-ray image is input to the learning model 12M without change. However, as in step S42 of FIG. 7, a process of extracting a region of the target site used to estimate the bone density of the proximal femur from the acquired X-ray image may be performed, and the X-ray image of the extracted region may be input to the learning model 12M. The target site used to estimate the bone density of the proximal femur may be the clavicle, the thoracic vertebra, etc., in addition to the rib.

The control unit 11 inputs the predicted DRR image of the rib generated in step S62 to the bone density estimation learning model 12M1 and estimates the bone density of the proximal femur of the subject (S63). Note that the control unit 11 acquires the bone density of the proximal femur as output information of the bone density estimation learning model 12M1. The control unit 11 calculates a numerical value indicating results of young adult comparison (YAM) and age-matched comparison from the estimated bone density (BMD) (S64). Then, the control unit 11 stores test results in, for example, electronic medical record data (S65), generates a screen displaying the test results, and outputs the screen to the display unit 15 (S66). Here, the control unit 11 generates, for example, a test result screen illustrated in FIG. 13. The screen illustrated in FIG. 13 displays subject identification information, a chest X-ray image, and an imaging date and time thereof, and displays, as test results of the bone density based on the chest X-ray image, a predicted DRR image of the rib, a target site name in the predicted DRR image (rib in FIG. 13), bone density of the proximal femur estimated from the predicted DRR image, young adult mean, and age-matched comparison. Here, when comments corresponding to the test results (bone density, young adult mean, or age-matched comparison) are stored in the storage unit 12, the control unit 11 may read the comments corresponding to the test results from the storage unit 12 and display the comments on the test result screen.

In this embodiment, a similar effect to that of the above-mentioned Embodiment 1 can be obtained. In addition, in this embodiment, the bone density of the proximal femur of the subject can be estimated from the chest X-ray image captured using the X-ray device. Therefore, in this embodiment, for example, when a chest X-ray image is captured in a health checkup, it is possible to estimate the bone density of the proximal femur of the subject from the chest X-ray image, and it is possible to more easily carry out a bone density measurement test at the time of other tests. For this reason, even for a subject for whom bone density measurement is not the purpose of the test, it is possible to diagnose the possibility of osteopenia or osteoporosis, and to perform early diagnosis and early treatment intervention.

In addition, in this embodiment, a predicted DRR image of a specific site (for example, a rib) generated from a chest X-ray image and bone density of the proximal femur estimated from the predicted DRR image can be presented to the doctor, etc. Therefore, the doctor can determine states of the rib and the proximal femur of the patient based on the predicted DRR image of the specific site and the estimated bone density of the proximal femur, and diagnose osteopenia or osteoporosis. Note that the target of bone density estimation is not limited to the proximal femur, and can be any site, and a capturing target of the X-ray image used for bone density estimation may be any site other than the site targeted for bone density estimation.

In this embodiment, the learning model 12M and the bone density estimation learning model 12M1 may be configured as one learning model. Specifically, for example, a learning model may be configured to receive input of the chest X-ray image and output the bone density of the proximal femur of the subject of the chest X-ray image. In addition, in this embodiment, instead of the bone density estimation learning model 12M1, it is possible to use a learning model trained to receive input of a predicted DRR image of a predetermined site (for example, a rib) generated from a chest X-ray image using the learning model 12M, generate a predicted DRR image of the proximal femur from the predicted DRR image, and output the predicted DRR image of the proximal femur. In this case, the DRR image of the proximal femur of the subject can be estimated from the predicted DRR image of the rib, and the bone density of the proximal femur can be estimated based on the estimated DRR image of the proximal femur. Furthermore, for example, it is possible to use a learning model trained to receive input of a chest X-ray image, and generate and output a predicted DRR image of the proximal femur of the subject of the chest X-ray image. In this case, the DRR image of the proximal femur of the subject can be estimated from the chest X-ray image, and thus the bone density of the proximal femur can be estimated based on the DRR image of the proximal femur. In this embodiment, it is possible to apply a modified example appropriately described in the above-mentioned Embodiment 1.

### (Embodiment 3)

In the above-mentioned Embodiment 1, a description has been given of a configuration in which a predicted DRR image of a target site (bone region such as the proximal femur) is generated from an X-ray image of the target site, and the bone density of the target site is estimated from the predicted DRR image. In this embodiment, a description will be given of an information processing apparatus that generates a predicted DRR image of a muscle region which is a target site (for example, a muscle region such as a lower abdomen or buttocks) from an X-ray image of the target site, and estimates muscle mass of the target site from the generated predicted DRR image. The information processing apparatus of this embodiment has a configuration similar to that of the information processing apparatus 10 of Embodiment 1, and thus a description of the configuration will be omitted. Note that the medical image DB 12a of this embodiment stores an X-ray image and a CT image of a site (muscle region) for which muscle mass is to be estimated in association with each other.

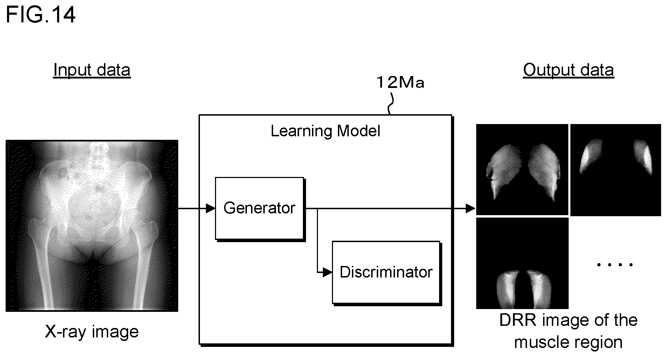

FIG. 14 is an explanatory view illustrating an overview of a learning model 12Ma of Embodiment 3. The learning model 12Ma of this embodiment has a similar configuration to that of the learning model 12M of Embodiment 1 illustrated in FIG. 2, and is generated by a similar training process. Note that, for example, the learning model 12Ma of this embodiment is trained using a frontal hip joint X-ray image (X-ray image of buttocks) and a DRR image of the muscle region of the buttocks generated from a CT image obtained by capturing the buttocks (lower abdomen) using the X-ray CT device as training data to output a DDR image (predicted DRR image, information related to the muscle mass) of the muscle region of the buttocks when an X-ray image of the buttocks is input. In an example illustrated in FIG. 14, the learning model 12Ma generates DRR images of a plurality of muscle regions such as gluteus maximus muscle, gluteus medius muscle, and hamstrings, but may be configured to generate a DRR image of any one of the muscle regions. For example, when there is a muscle region that can be used to diagnose sarcopenia, the learning model 12Ma may be generated to generate a DRR image of the corresponding muscle region.

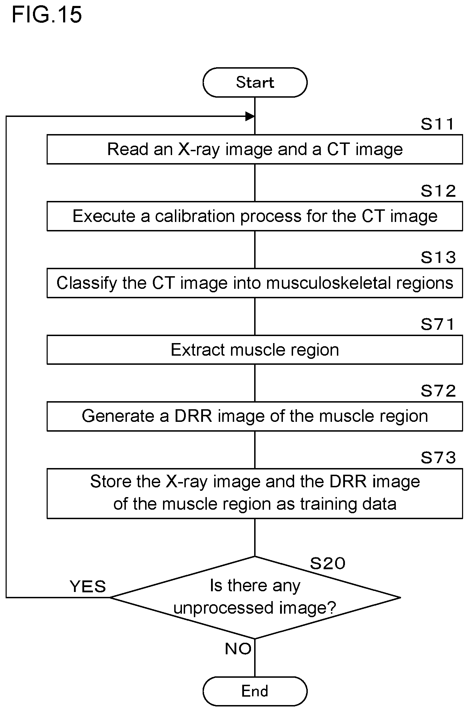

Hereinafter, a description will be given of a process of generating training data used to train the learning model 12Ma of this embodiment. FIG. 15 is a flowchart illustrating an example of a generation process procedure for training data of Embodiment 3. Processing illustrated in FIG. 15 is obtained by adding step S71 instead of step S14, deleting steps S15 to S16 and S18, and adding steps S72 and S73 instead of steps S17 and S19 in the processing illustrated in FIG. 4. A description of the same steps as those of FIG. 4 will be omitted.

The control unit 11 of the information processing apparatus 10 of this embodiment performs similar processing to that of steps S11 to S13 of FIG. 4. That is, the control unit 11 reads a pair of an X-ray image (frontal hip joint X-ray image) and a CT image from the medical image DB 12a, performs a luminance value calibration process on the read CT image, and then classifies each pixel in the CT image as a bone region, a muscle region, and another region (musculoskeletal region). In this way, as illustrated in (1) of FIG. 5, a musculoskeletal labeled image in which each pixel in the CT image is classified as each region can be acquired. In this embodiment, the control unit 11 extracts muscle region data from the CT image based on the musculoskeletal labeled image (S71). The control unit 11 extracts muscle region data (CT image) for each muscle type.

The control unit 11 generates a DRR image of the muscle region by projecting each pixel of the CT image in a predetermined direction from the extracted muscle region data (CT image) (S72). For example, when the X-ray image is obtained by receiving radiation passing through the subject from a back to a front, the control unit 11 generates a DRR image by projecting each pixel of the CT image of the muscle region in a direction from the back to the front of the subject. Here, the control unit 11 sets an integrated value of each pixel value (luminance value, voxel value) arranged in a predetermined direction in the CT image as each pixel value of the DRR image. Therefore, in this embodiment, each pixel value in the DRR image of the muscle region corresponds to muscle density (muscle mass) at each position. Note that the control unit 11 may align a capturing target (here, each muscle) in each image in the X-ray image acquired in step S11 and the CT image of the muscle region extracted in step S71, and generate a DRR image obtained by projecting each pixel of the CT image after alignment in the same direction as the capturing direction of the X-ray image.

The control unit 11 associates the X-ray image acquired in step S11 with the DRR image of the muscle region generated in step S72 and stores the images in the training DB 12b as training data (S73). Thereafter, the control unit 11 proceeds to the process of step S20. Through the above-mentioned processing, training data used to train the learning model 12Ma of this embodiment can be generated based on the X-ray image and the CT image stored in the medical image DB 12a and accumulated in the training DB 12b. By using the training data generated in this way, in this embodiment, the learning model 12Ma can be generated by a process similar to the training process illustrated in FIG. 6.

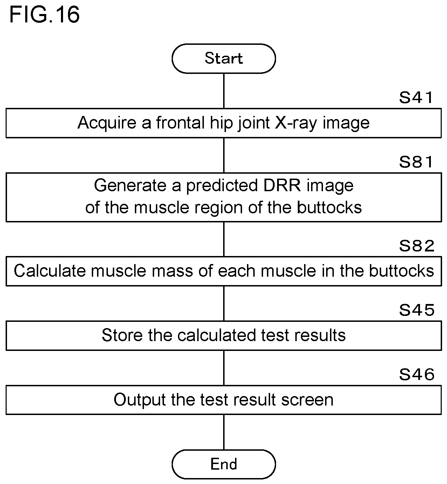

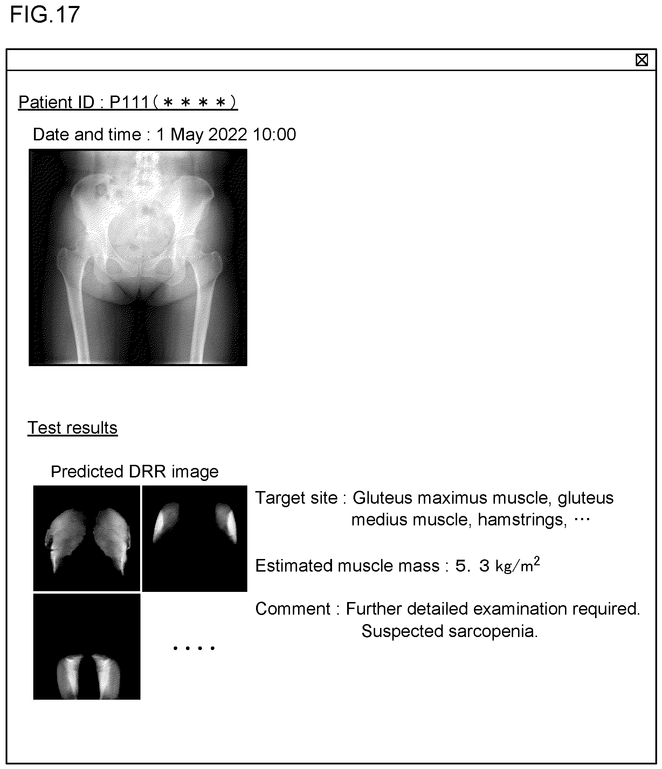

Next, a description will be given of a process of estimating the muscle mass of the buttocks of the subject from the frontal hip joint X-ray image of the subject using the learning model 12Ma. FIG. 16 is a flowchart illustrating an example of an estimation process procedure for muscle mass, and FIG. 17 is an explanatory view illustrating a screen example. Processing illustrated in FIG. 16 is obtained by deleting step S42, and adding steps S81 to S82 instead of steps S43 to S44 in the processing illustrated in FIG. 7. A description of the same steps as those of FIG. 7 will be omitted.

The control unit 11 of the information processing apparatus 10 acquires a frontal hip joint X-ray image of the subject (S41). The control unit 11 generates a predicted DRR image of the muscle region in the X-ray image based on the frontal hip joint X-ray image (S81). Specifically, the control unit 11 inputs the frontal hip joint X-ray image to the learning model 12Ma, and acquires the predicted DRR image of the muscle region of the buttocks in the X-ray image as output information from the learning model 12Ma. Then, the control unit 11 calculates muscle mass of each muscle in the buttocks from the generated predicted DRR image of the muscle region (S82). In this embodiment, each pixel value of the predicted DRR image is a value corresponding to muscle density, and the control unit 11 calculates an average value of each pixel value in the predicted DRR image to calculate the muscle density in the muscle region. In addition, the control unit 11 calculates the muscle mass in the muscle region based on the calculated muscle density and the volume of the muscle region. Note that the control unit 11 may calculate the muscle mass for each pixel based on each pixel value in the predicted DRR image, and may calculate the muscle mass in the muscle region by integrating the muscle masses corresponding to each pixel. Furthermore, the control unit 11 may predict the muscle mass of the entire body of the subject based on the muscle mass in each muscle region. For example, by registering the muscle mass of each muscle of the subject, such as the gluteus maximus muscle, gluteus medius muscle, and hamstrings, in association with the muscle mass of the entire body of the subject, the muscle mass of the entire body of the subject can be predicted from the muscle mass of each muscle estimated from the predicted DRR image. In addition, the control unit 11 may predict muscle mass of upper limbs or muscle mass of lower limbs, etc. of the subject based on the muscle mass in each muscle region.

The control unit 11 stores test results including the calculated muscle density and muscle mass of each muscle in, for example, the electronic medical record data (S45), generates a test result screen illustrated in FIG. 17, and outputs the test result screen to the display unit 15 (S46). The screen illustrated in FIG. 17 displays identification information of the subject, the frontal hip joint X-ray image, and an imaging date and time thereof. In addition, the screen illustrated in FIG. 17 displays the predicted DRR image of each muscle, the target site name in the predicted DRR image (gluteus maximus muscle, gluteus medius muscle, and hamstrings in FIG. 17), and the muscle mass estimated from the predicted DRR image as muscle mass test results based on the frontal hip joint X-ray image. In addition, when comments to be presented to the doctor, etc. are stored in the storage unit 12 in association with muscle density or muscle mass, the control unit 11 may read the comments corresponding to the calculated test result (muscle density or muscle mass) from the storage unit 12 and display the comments on the test result screen as illustrated in FIG. 17.

By the above-mentioned processing, in this embodiment, it is possible to estimate the muscle mass of the muscle region in the frontal hip joint X-ray image taken using the X-ray device from the frontal hip joint X-ray image. Further, in this embodiment, it is possible to present to the doctor, etc. the predicted DRR image of the muscle region generated from the frontal hip joint X-ray image and the muscle mass estimated from the predicted DRR image. Therefore, the doctor can determine a muscle condition of the patient based on the predicted DRR image and the estimated muscle mass. In this embodiment, the learning model 12Ma automatically extracts features of the imaging state of the muscle region in the X-ray image to generate the predicted DRR image of the muscle region, so that it is possible to estimate the muscle mass by simply capturing an image using the X-ray device without performing a test using the DXA device, etc. Therefore, it is possible to estimate the muscle mass of the target site from an X-ray image captured during a health checkup or at a small clinic, and thus it is possible to easily perform a muscle mass measurement test. Therefore, it is possible to perform early diagnosis of sarcopenia, which is a decrease in muscle mass and muscle strength due to aging, and it is expected to prevent and suppress onset and progression of sarcopenia and contribute to extension of healthy life expectancy.

In this embodiment, it is possible to realize the learning model 12Ma that can generate the predicted DRR image of the muscle region in the X-ray image with high accuracy from the X-ray image by training using a small amount of training data. Therefore, in this embodiment, it is possible to predict muscle mass and muscle density from the X-ray image to the same extent as that of the measurement results using the DXA device, and workload in a collection process and a training process for the training data can be reduced.

In this embodiment, a description has been given of a configuration in which the predicted DRR image of the muscle region of the buttocks (lower abdomen) is generated from the X-ray image of the buttocks using the learning model 12Ma, and the muscle density and the muscle mass of the buttocks are estimated from the predicted DRR image. The site targeted for estimation of the muscle density and the muscle mass may be the buttocks, as well as the upper limbs, the lower limbs, the chest, the abdomen, the entire body, etc. For other sites, training data and a learning model are generated by similar processing, and it is possible to estimate the muscle density and the muscle mass using the learning model.

In this embodiment, similar effects to those of the above-mentioned Embodiments 1 and 2 are obtained. In addition, in this embodiment, the muscle mass of the target site can be estimated from the X-ray image of the target site captured using the X-ray device. Therefore, it is easier to perform a muscle mass measurement test, so that even for a subject who is not worried about muscle mass, it is possible to diagnose a possibility of a disease such as sarcopenia, making it possible to diagnose sarcopenia early and to intervene in treatment early. In addition, the modified examples described in the above-mentioned Embodiments 1 and 2 can be appropriately applied to this embodiment as well.

### (Embodiment 4)

A description will be given of an embodiment combining the configuration of Embodiment 1 and the configuration of Embodiment 2. That is, in this embodiment, a description will be given of an information processing apparatus that generates a predicted DRR image of a target site from an X-ray image of the target site, estimates bone density of the target site based on the generated predicted DRR image, and estimates bone density of a site different from the target site. The information processing apparatus of this embodiment has a configuration similar to that of the information processing apparatus 10 of each of Embodiments 1 and 2, and thus a description of the configuration will be omitted. That is, the information processing apparatus 10 of this embodiment has a learning model 12M that generates a predicted DRR image of a target site from an X-ray image of the target site, and a bone density estimation learning model 12M1 that estimates bone density of a site different from the target site from the DRR image of the target site. In the following example, the learning model 12M is a model that generates a predicted DRR image of a proximal femur from an X-ray image of the proximal femur, and the bone density estimation learning model 12M1 is a model that estimates bone density of a rib from a DRR image of the proximal femur. However, the learning models 12M and 12M1 are not limited to such configurations, and a plurality of types of models may be prepared for each of the learning models 12M and 12M1. For example, as the learning model 12M, a model that generates (predicts) a DRR image of a pelvis from an X-ray image of the pelvis, a model that generates a DRR image of a rib, a clavicle, or a thoracic vertebra from an X-ray image of a chest, etc. may be further prepared. In addition, as the bone density estimation learning model 12M1, a model that estimates bone density of a rib, a clavicle, a thoracic vertebra, a lumbar vertebra, a cervical vertebra, a hand bone, or a foot bone from a DRR image of a proximal femur, a pelvis, or a femur, a model that estimates bone density of a proximal femur, a lumbar vertebra, a cervical vertebra, a hand bone, or a foot bone from a DRR image of a rib, a clavicle, or a thoracic vertebra, etc. may be prepared.

The information processing apparatus 10 of this embodiment can perform the processing illustrated in FIGS. 11 and 6, generates training data for training the learning models 12M and 12M1 by the processing illustrated in FIG. 11, and performs a process of training the learning models 12M and 12M1 by the processing illustrated in FIG. 6.

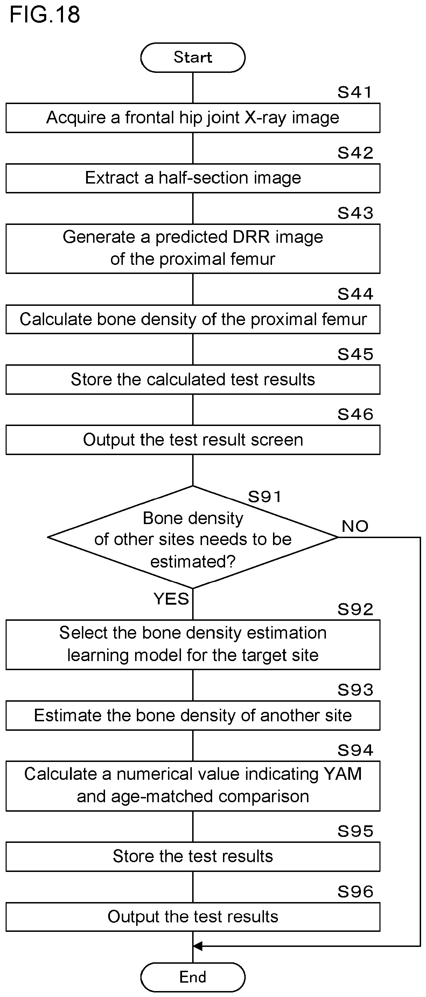

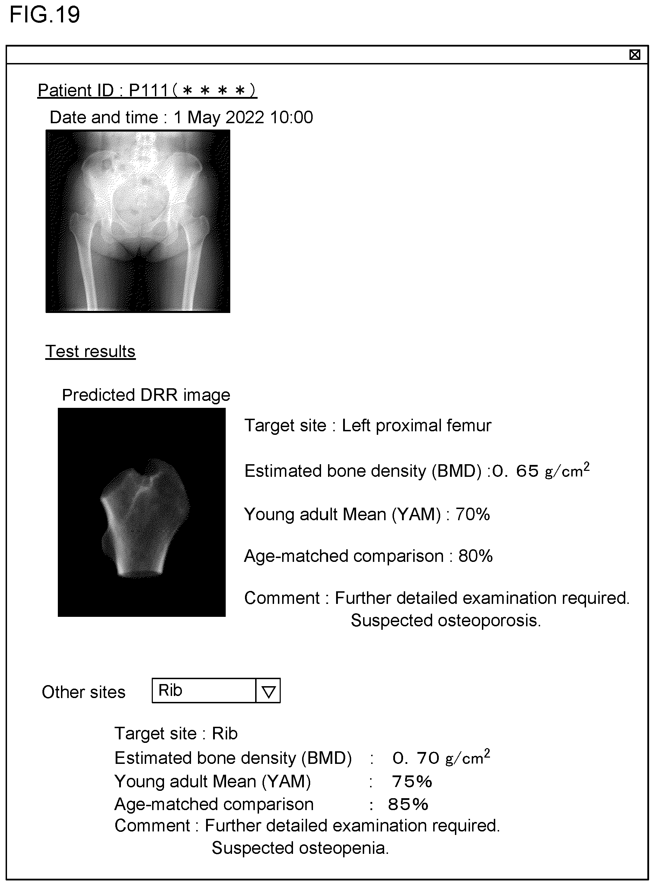

Next, a description will be given of a process of estimating bone density of a proximal femur and bone density of a rib of a subject from a frontal hip joint X-ray image of the subject using the learning models 12M and 12M1. FIG. 18 is a flowchart illustrating an example of an estimation process procedure for bone density of Embodiment 4, and FIG. 19 is an explanatory view illustrating a screen example. Processing illustrated in FIG. 18 is obtained by adding steps S91 to S96 after step S46 in the processing illustrated in FIG. 7. A description of the same steps as those of FIG. 7 will be omitted. The control unit 11 of the information processing apparatus 10 executes the same processing as that of steps S41 to S46 of FIG. 7. In this way, the information processing apparatus 10 estimates the bone density of the proximal femur of the subject from the frontal hip joint X-ray image of the subject, and displays the test result screen as illustrated in FIG. 8.

The control unit 11 determines whether or not bone density of a site other than the capturing target of the X-ray image needs to be estimated (S91). For example, as illustrated in FIG. 19, the test result screen is provided with an input field for selecting a site other than the capturing target of the X-ray image, and the control unit 11 determines that the bone density of the input site needs to be estimated when another site is input through the input field. Note that, in the example illustrated in FIG. 19, a pull-down menu for selecting any site is provided in the input field, and when the doctor, etc. desires to check the bone density of another site, the doctor selects the desired site through the pull-down menu. When the control unit 11 determines not to estimate the bone density of another site (S91: NO), that is, when another site is not input in the input field, the process ends.

When determining to estimate the bone density of another site (S91: YES), the control unit 11 selects the bone density estimation learning model 12M1 for the target site based on the site input to the input field (S92). Here, since the predicted DRR image of the proximal femur is generated in step S43 and the rib is selected as another site targeted for bone density estimation, the control unit 11 selects the bone density estimation learning model 12M1 for estimating the bone density of the rib from the DRR image of the proximal femur. Then, the control unit 11 inputs the predicted DRR image of the proximal femur generated in step S43 to the selected bone density estimation learning model 12M1, and estimates the bone density of the rib (another site) of the subject based on output information from the bone density estimation learning model 12M1 (S93). Thereafter, the control unit 11 executes the same processing as that of steps S64 to S65 of FIG. 12 (S94 to S95), and outputs the obtained test results to a currently displayed test result screen (S96). In this way, as illustrated in FIG. 19, in addition to the bone density of the proximal femur estimated from the frontal hip joint X-ray image, it is possible to display the bone density of the rib of the subject.

In this embodiment, the same effects as those of the above-mentioned embodiments can be obtained. In addition, in this embodiment, not only the bone density of the target site but also the bone density of other sites can be estimated and presented from the X-ray image obtained by capturing the target site. Therefore, in this embodiment, not only the bone density of the site of the capturing target of the X-ray image but also the bone density of another site can be estimated based on an X-ray image captured during a medical checkup or hospital visit. Therefore, even when capturing an X-ray image of a site other than a site where the bone density is to be confirmed, the bone density of the site where the bone density is to be confirmed can be predicted, which may lead to early diagnosis and early treatment of osteopenia or osteoporosis. Further, in this embodiment, the modified examples described in each of the above-mentioned embodiments can be applied as appropriate.

### (Embodiment 5)

A description will be given of an embodiment combining the configuration of Embodiment 1 and the configuration of Embodiment 3. That is, in this embodiment, a description will be given of an information processing apparatus that estimates bone density in a bone region of a target site from an X-ray image of the target site and estimates muscle mass in the muscle region of the target site. The information processing apparatus of this embodiment has a configuration similar to that of the information processing apparatus 10 of each of Embodiments 1 and 3, and thus a description of the configuration will be omitted. That is, the information processing apparatus 10 of this embodiment has a learning model 12M that generates a predicted DRR image of a bone region of a target site from an X-ray image of the target site, and a learning model 12Ma that generates a predicted DRR image of a muscle region of a target site from an X-ray image of the target site. In the following example, the learning model 12M is a model that generates a predicted DRR image of a proximal femur from a frontal hip joint X-ray image, and the learning model 12Ma is a model that generates a predicted DRR image of a muscle region of buttocks from a frontal hip joint X-ray image. However, the learning models 12M and 12Ma are not limited to such a configuration, and a plurality of types of models may be prepared for each of the learning models 12M and 12Ma. For example, a model that generates (predicts) a DRR image of a rib, a clavicle, or a thoracic vertebra from a chest X-ray image may be prepared as the learning model 12M, and a model that generates a DRR image of a pectoralis major, a pectoralis minor, a subclavius, a serratus anterior, or an intercostal muscle from a chest X-ray image may be prepared as the learning model 12Ma.

The information processing apparatus 10 of this embodiment is capable of executing the processing illustrated in FIGS. 4, 6, and 15, and generates training data for training the learning model 12M by the processing illustrated in FIG. 4, generates training data for training the learning model 12Ma by the processing illustrated in FIG. 15, and executes a process of training the learning models 12M and 12Ma by the processing illustrated in FIG. 6.

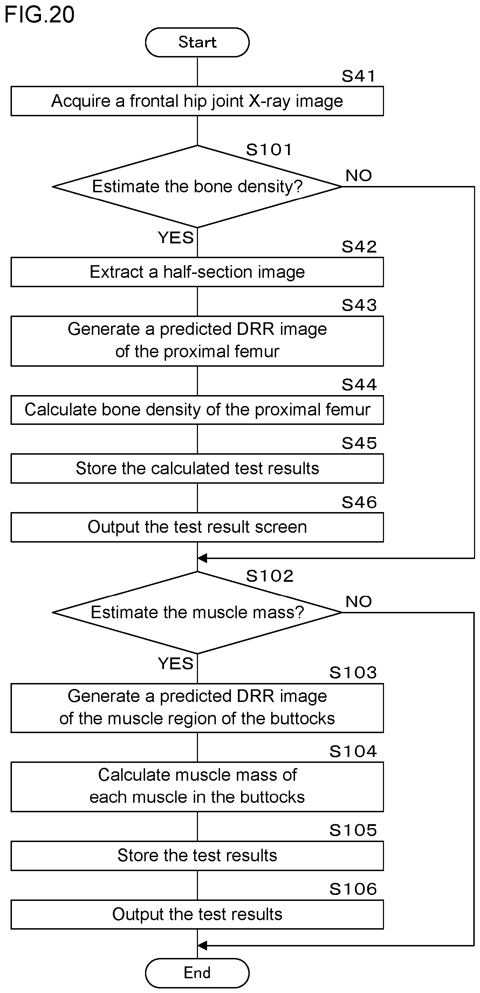

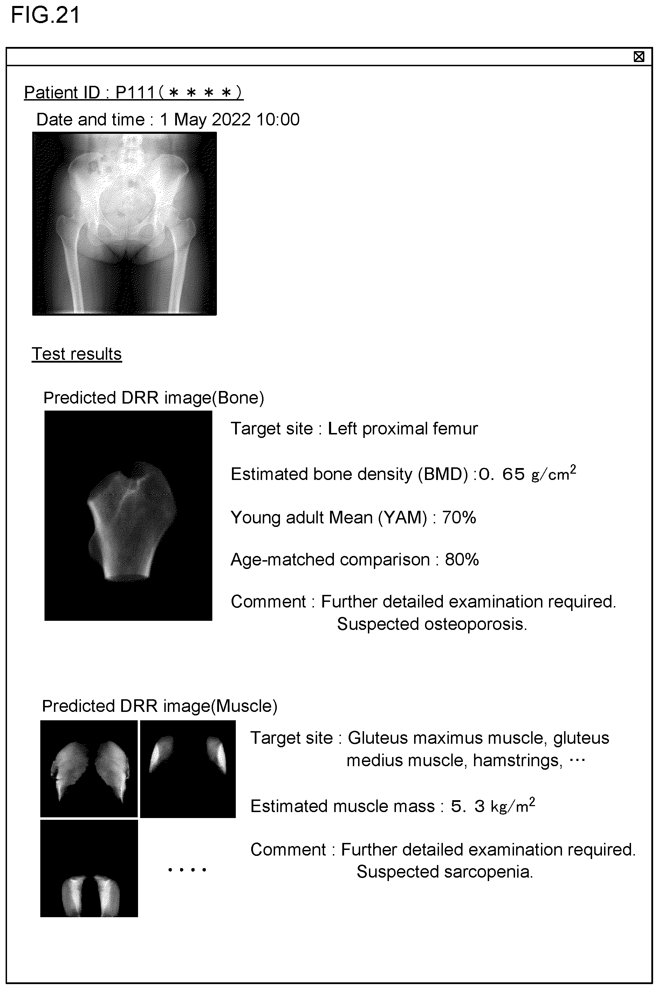

Next, a description will be given of a process of estimating the bone density of the proximal femur and the muscle mass of the buttocks of the subject from a frontal hip joint X-ray image of the subject using the learning models 12M and 12Ma. FIG. 20 is a flowchart illustrating an example of an estimation process procedure for bone density and muscle mass of Embodiment 5, and FIG. 21 is an explanatory view illustrating a screen example. Processing illustrated in FIG. 20 is obtained by adding step S 101 between steps S41 and S42, and adding steps S102 to S106 after step S46 in the processing illustrated in FIG. 7. A description of the same steps as those of FIG. 7 will be omitted.

After acquiring the frontal hip joint X-ray image of the subject (S41), the control unit 11 of the information processing apparatus 10 determines whether or not to estimate the bone density of the proximal femur based on the acquired X-ray image (S101). For example, the doctor, etc. designates via a menu screen (not illustrated) whether to estimate the bone density of the bone region or estimate the muscle mass of the muscle region for the capturing target in the X-ray image. When estimation of the bone density of the bone region is designated via the menu screen, the control unit 11 determines to estimate the bone density of the proximal femur (S101: YES) and executes similar processing to that of steps S42 to S46 of FIG. 7. In this way, the information processing apparatus 10 estimates the bone density of the proximal femur of the subject from the frontal hip joint X-ray image of the subject, and displays a test result screen as illustrated in FIG. 8.

When determining not to estimate the bone density of the proximal femur (S101: NO), the control unit 11 proceeds to processing of step S102 and determines whether or not to estimate the muscle mass of the muscle region of the buttocks, which is the capturing target of the acquired X-ray image (S102). When estimation of the muscle mass of the muscle region is not designated via the menu screen, the control unit 11 determines not to estimate the muscle mass of the buttocks (S102: NO) and ends the process. On the other hand, when estimation of the muscle mass of the muscle region is designated via the menu screen, the control unit 11 determines to estimate the muscle mass of the buttocks (S102: YES), and executes similar processing to that of as steps S81 to S82 and S45 of FIG. 16 (S103 to S105). In this way, the information processing apparatus 10 estimates the muscle mass of the muscle region of the buttocks of the subject from the frontal hip joint X-ray image of the subject, and outputs the obtained test results to a currently displayed test result screen (S106). Therefore, as illustrated in FIG. 21, in addition to the bone density of the proximal femur estimated from the frontal hip joint X-ray image, it is possible to display the muscle mass of the buttocks of the subject. By the above-mentioned processing, the bone density of the bone region of the target site and the muscle mass of the muscle region can be estimated from the X-ray image of the target site, and whether any one or both of the bone density and the muscle mass needs to be targeted for estimation can be appropriately switched according to designation by the user such as the doctor.

In this embodiment, a similar effect to that in each of the above-mentioned embodiments can be obtained. In addition, in this embodiment, not only the bone density of the bone region of the target site but also the muscle mass of the muscle region of the target site can be estimated and presented from the X-ray image obtained by capturing the target site. Therefore, a bone condition and a muscle condition in the capturing target can be checked, thereby allowing early diagnosis of osteopenia or osteoporosis, as well as early diagnosis of a disease such as sarcopenia. In this embodiment, the modified examples described in each of the above-mentioned embodiments can be applied as appropriate.

### (Embodiment 6)

A description will be given of a process of aligning, by an information processing apparatus, a bone region in an X-ray image (target site or region of interest) and a bone region (target site or region of interest) in a CT image in the X-ray image (plain X-ray images) and the CT image obtained by capturing the same capturing target of the same subject in the above-mentioned Embodiments 1 to 5. The information processing apparatus of this embodiment has a similar configuration to that of the information processing apparatus 10 of each of Embodiments 1 to 5, and thus a description of the configuration will be omitted.

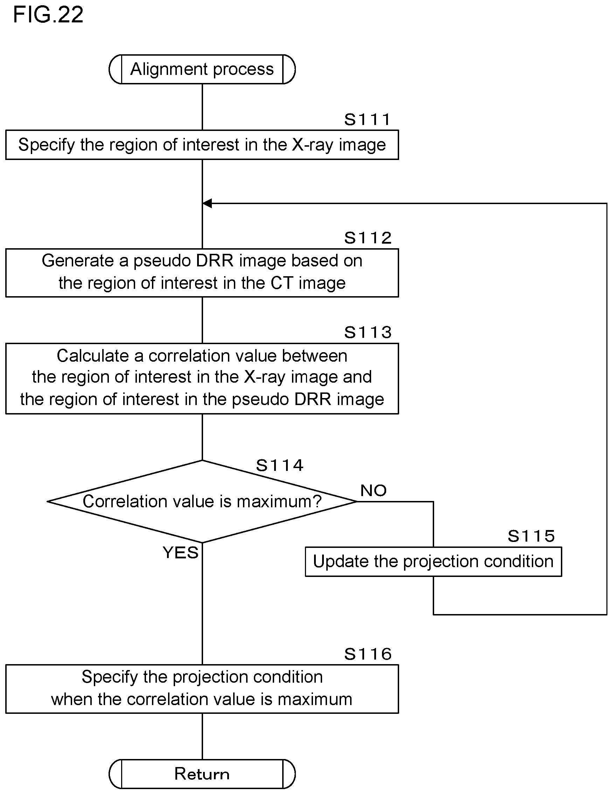

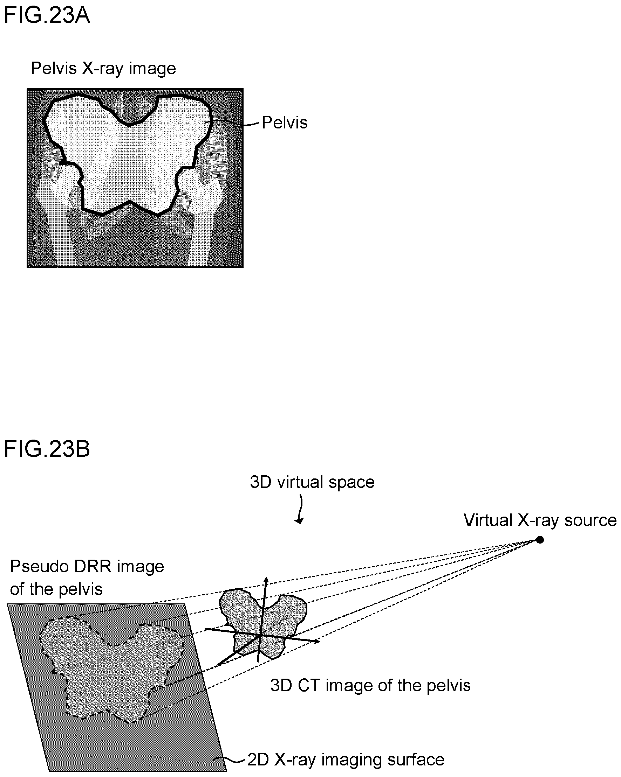

FIG. 22 is a flowchart illustrating an example of an alignment process procedure, and FIG. 23A to FIG. 24B are explanatory views of an alignment process. The alignment process illustrated in FIG. 22 is the processing of step S16 of FIG. 4 and FIG. 11. Therefore, in this embodiment, the control unit 11 of the information processing apparatus 10 executes processing of FIG. 22 after the processing of step S15 of FIG. 4 and FIG. 11, and then executes the processing of step S17 of FIG. 4 and FIG. 11. In the following, a pelvis is taken as the region of interest, and alignment between the pelvis in the frontal hip joint X-ray image and the pelvis in the CT image is described as an example. However, the bone region used for alignment is not limited to the region of interest. Any bone region captured in the X-ray image and the CT image can be used for the alignment. For example, in the frontal hip joint X-ray image, in addition to the pelvis, a femur, a proximal femur, etc. may be used for alignment.

After the processing of step S15 of FIG. 4 and FIG. 11, the control unit 11 specifies the region of interest (here, the pelvis) in the X-ray image (here, the frontal hip joint X-ray image) acquired in step S11 (S111). A process of specifying the region of interest in the X-ray image can be performed, for example, by pattern matching using a template that indicates a shape of the region of interest, or can be performed, for example, using a learning model machine-trained to output the region of interest in the X-ray image when the X-ray image is input. In this way, for example, a pelvis region indicated by a solid line in the X-ray image illustrated in FIG. 23A can be specified.

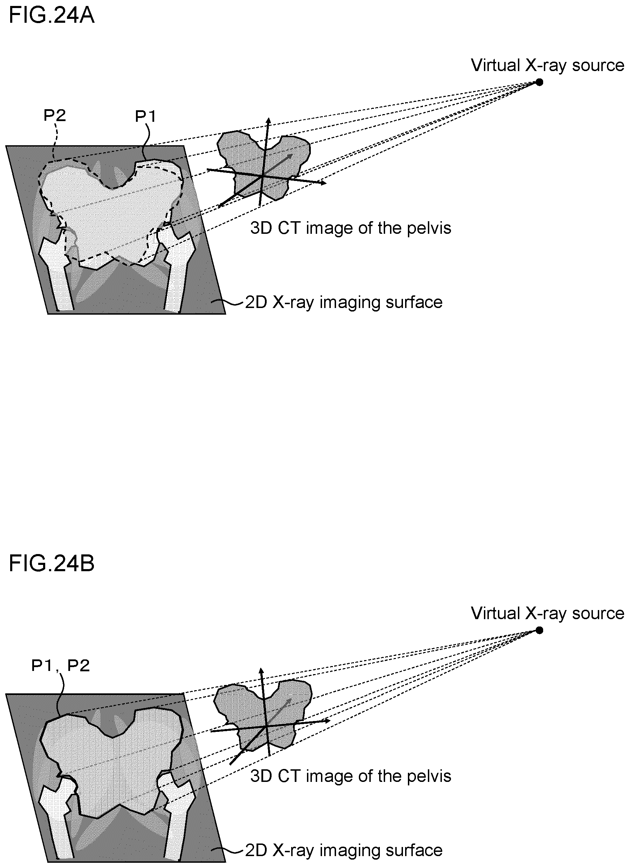

Next, the control unit 11 generates a pseudo DRR image of a region of interest based on the region of interest in the CT image extracted in step S15 (S112). Here, the control unit 11 generates a pseudo DRR image of the pelvis. Specifically, as illustrated in FIG. 23B, the control unit 11 disposes the region of interest in the CT image (3D CT image of the pelvis) in a 3D virtual space of an X-ray imaging system under a predetermined projection condition (position and angle relative to a virtual X-ray source), and generates a pseudo DRR image (projection image) by projecting the region of interest from the virtual X-ray source onto a 2D X-ray imaging surface. Here, since an imaging condition (for example, a posture, a bending angle of a joint, etc. of the subject) is different between when the X-ray image is captured and when the CT image is captured, a contour of the region of interest in the pseudo DRR image generated from the CT image does not match a contour of the region of interest in the X-ray image. In FIG. 24A, a contour P1 of the region of interest in the X-ray image is indicated by a solid line, and a contour P2 of the region of interest in the pseudo DRR image is indicated by a dashed line. In this embodiment, the control unit 11 updates a projection condition of the region of interest in the CT image to specify a projection condition that maximizes a correlation value between the contour P1 of the region of interest in the X-ray image and the contour P2 of the region of interest in the pseudo DRR image. With such a projection condition, it is possible to obtain a pseudo DRR image in which the region of interest in the pseudo DRR image is accurately aligned with the region of interest in the X-ray image, as illustrated in FIG. 24B.

Therefore, the control unit 11 calculates a correlation value between the contour of the region of interest in the X-ray image specified in step S111 and the contour of the region of interest in the pseudo DRR image generated in step S112 (S113), and determines whether or not the calculated correlation value is maximum (S114). When determining that the correlation value is not maximum (S114: NO), the control unit 11 updates the projection condition when generating the pseudo DRR image from the CT image of the region of interest (S115), and repeats the processing of steps S112 to S114 under the updated projection condition. The control unit 11 repeats the processing of steps S112 to S115 until determining that the calculated correlation value is maximum, and when determining that the correlation value is maximum (S114: YES), that is, when a pseudo DRR image having the maximum correlation value can be generated, the control unit 11 specifies a projection condition at this time (S116).

In this embodiment, the control unit 11 can perform the processing of steps S112 to S115 using, for example, a method described in an article entitled "3D-2D registration in mobile radiographs: algorithm development and preliminary clinical evaluation" by the present inventor, Yoshito Otake et al. The article discloses a method of defining achievement of alignment between a contour in a DRR image (here, pseudo DRR image) of a target site (bone region, here, pelvis) generated from a 3D region of the target site in a CT image and a contour of a target site in an actual X-ray image as "maximization of as a correlation between gray-scale gradient intensity images of the X-ray image and the DRR image, respectively", and obtaining arrangement that maximizes the correlation (projection condition, specifically, a three-dimensional position and angle of the target site relative to an imaging system) using a covariance matrix adaptation evolution strategy (CMA-ES). Therefore, the control unit 11 can use the CMA-ES to specify a pseudo DRR image in which a correlation value between the contour of the region of interest in the X-ray image and the contour of the pseudo DRR image is maximized, thereby specifying a projection condition for the specified pseudo DRR image. Under such a projection condition, as illustrated in FIG. 24B, it is possible to generate a pseudo DRR image of the region of interest in which the contour P2 of the region of interest in the pseudo DRR image is aligned with the contour P1 of the region of interest in the X-ray image with high accuracy. Note that, in step S114, the control unit 11 may be configured to determine whether or not the calculated correlation value is equal to or greater than a predetermined value, and to proceed to the processing of step S116 when it is determined that the calculated correlation value is equal to or greater than the predetermined value.

Thereafter, the control unit 11 proceeds to the processing of step S17 of FIG. 4 and FIG. 11, and generates a DRR image by projecting the CT image of the region of interest in a projection direction according to the projection condition specified in step S116 (S17). Specifically, the control unit 11 calculates an integrated value of a pixel value (luminance value, voxel value) of each pixel aligned in the projection direction according to the projection condition specified in step S116 in the CT image of the region of interest, and sets the calculated integrated value as each pixel value of the DRR image of the region of interest. In this way, a DRR image of the target site is generated under the same projection condition as the imaging condition (position and angle relative to the X-ray source) of the target site (region of interest) in the X-ray image, and the DRR image of the target site accurately aligned with the target site in the X-ray image is obtained. Thereafter, the control unit 11 can execute the processing of steps S18 to S19 of FIG. 4 to associate a half-section image of the X-ray image (frontal hip joint X-ray image) with a DRR image of the region of interest projected in the same direction as capturing direction of the X-ray image, thereby generating training data for training of the learning model 12M. In addition, by executing S19 and S52 of FIG. 11, the control unit 11 can generate training data for training the learning model 12M by associating the X-ray image with the DRR image of the region of interest projected in the same direction as the capturing direction of the X-ray image, and generate training data for training the bone density estimation learning model 12M1 by associating the DRR image of the region of interest with the bone density of the region of interest acquired in step S51.

By the above-mentioned processing, training data in which the X-ray image of the target site (the region of interest which is the bone region) and the DRR image of the target site highly accurately aligned with the target site in the X-ray image are associated with each other is generated and stored in the training DB 12b. Note that the bone region is a hard tissue, and thus is considered not to be deformed when the X-ray image is captured and when the CT image is captured. Therefore, by using an alignment process based on optimization of a condition (projection condition) when generating the DRR image from the CT image are optimized as in this embodiment, the contour of the target site (bone region) in the X-ray image can be accurately aligned with the contour of the target site in the DRR image generated from the CT image. In addition, the information processing apparatus 10 of this embodiment can execute the processing illustrated in FIG. 6, and by executing the process of training the learning model 12M according to the processing illustrated in FIG. 6 using the training data generated as described above, it is possible to realize the learning model 12M capable of predicting a DRR image with high accuracy from an X-ray image. Furthermore, the information processing apparatus 10 of this embodiment can execute the processing illustrated in FIGS. 7, 12, 18, and 20, and estimate the bone density of the target site with high accuracy by the DRR image predicted with high accuracy from the X-ray image by using the learning model 12M generated as described above, and a highly accurate test result can be obtained.

The above-mentioned processing has a configuration in which the region of interest in the X-ray image and the CT image is aligned based on the region of interest extracted from the CT image in step S15 in the processing illustrated in FIG. 4. However, the present disclosure is not limited to this configuration. For example, the bone region in the X-ray image and the CT image may be aligned based on the bone region extracted from the CT image in step S14. In this case, the control unit 11 of the information processing apparatus 10 executes the alignment process illustrated in FIG. 22 after the processing of step S14 of FIG. 4, and executes the processing of steps S15 and S17 based on the CT image after the alignment process. Specifically, the control unit 11 extracts data of a region of interest (for example, left proximal femur) from the CT image after the alignment process (S15), and generates a DRR image projected from the CT image of the extracted region of interest in a projection direction according to the projection condition specified in step S116 (S17). Note that, in the processing illustrated in FIG. 22 here, the bone region extracted in step S14 is treated as a region of interest, and processing of steps S111 to S116 is executed. Thereafter, the control unit 11 executes processing of steps S18 to S19. Even in such processing, training data, in which an X-ray image of a target site (region of interest) and a DRR image of the target site highly accurately aligned with the target site in the X-ray image are associated with each other, is generated and stored in the training DB 12b. By executing the process of training the learning model 12M using the training data generated in this way, it is possible to realize the learning model 12M capable of predicting a DRR image with high accuracy from an X-ray image. In addition, by using the learning model 12M generated in this way, it is possible to predict a DRR image with high accuracy from an X-ray image, and it is possible to estimate the bone density of the target site with high accuracy using the DRR image predicted with high accuracy.

In this embodiment, processing other than the above-mentioned alignment process is similar to that in each of the above-mentioned embodiments, and a similar effect to that in each of the above-mentioned embodiments can be obtained. In addition, in this embodiment, spatial alignment between the target site in the X-ray image and the target site in the CT image can be performed with high accuracy. Therefore, by using, as training data, such an X-ray image and a DRR image generated from a CT image aligned with the X-ray image with high accuracy, it is possible to realize the learning model 12M that predicts the DRR image from the X-ray image with high accuracy without requiring a large number of cases (training data). In addition, in this embodiment, the modified examples appropriately described in each of the above-mentioned embodiments can be applied.

### (Embodiment 7)

The above-mentioned configuration of Embodiment 6 is applied to the above-mentioned Embodiments 1 and 3, and a description will be given of a process in which an information processing apparatus aligns the capturing target (target site or region of interest) in the X-ray image and the CT image based on the bone region in the X-ray image and the CT image and generates training data used to train the learning models 12M and 12Ma based on the CT image after alignment. The information processing apparatus of this embodiment has a similar configuration to that of the information processing apparatus 10 of each of Embodiments 1 and 3, and thus a description of the configuration will be omitted. That is, the information processing apparatus 10 of this embodiment has the learning models 12M and 12Ma.

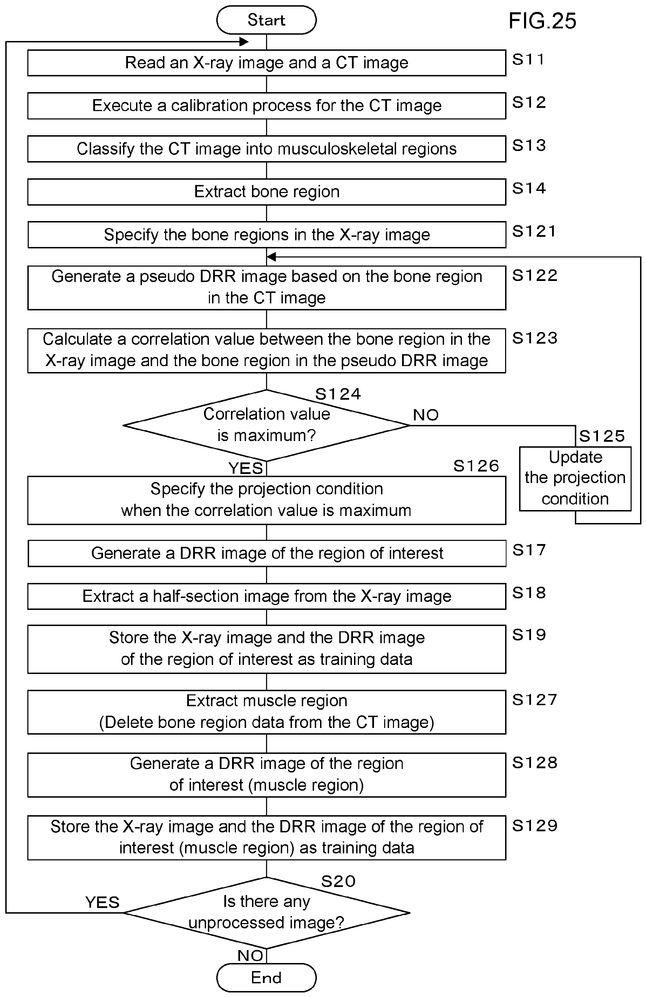

FIG. 25 is a flowchart illustrating an example of a generation process procedure for training data of Embodiment 7. Processing illustrated in FIG. 25 is obtained by adding steps S121 to S126 instead of steps S15 and S16 and adding steps S127 to S129 between the steps S19 and S20 in the processing illustrated in FIG. 4. A description of the same steps as those of FIG. 4 will be omitted.

The control unit 11 of the information processing apparatus 10 of this embodiment executes the processing of steps S11 to S14 of FIG. 4. Note that, in step S14, the control unit 11 extracts data of any bone region from the CT image based on a musculoskeletal labeled image generated from the CT image. For example, in the CT image illustrated in FIG. 5, the control unit 11 may extract bone regions of a pelvis, a femur, a proximal femur, etc., or a plurality or all of these bone regions. After extracting the bone regions in the CT image, the control unit 11 executes processing similar to that of steps S111 to S116 of FIG. 22 based on the bone regions extracted from the CT image (S121 to S126). Specifically, the control unit 11 specifies the same bone regions in the X-ray image as the bone regions extracted from the CT image (S121).

Next, the control unit 11 generates a pseudo DRR image of the bone regions based on the bone regions in the CT image extracted in step S14 (S122). Then, the control unit 11 calculates a correlation value between a contour of the bone regions in the X-ray image specified in step S121 and a contour of the bone regions in the pseudo DRR image generated in step S122 (S123), and determines whether or not the calculated correlation value is maximum (S124). When determining that the correlation value is not maximum (S124: NO), the control unit 11 updates a projection condition when generating the pseudo DRR image from the CT image of the bone regions (S125) and repeats the processing of steps S122 to S124 with the updated projection condition. The control unit 11 repeats the processing of steps S122 to S125 until determining that the calculated correlation value is maximum, and when determining that the correlation value is maximum (S124: YES), that is, when a pseudo DRR image having a maximum correlation value can be generated, the control unit 11 specifies a projection condition at this time (S126). In this embodiment, the control unit 11 specifies a projection condition in the pseudo DRR image maximizing the correlation value between the contour of the bone regions in the X-ray image and the contours of the pseudo DRR image by using the CMA-ES in the processing of steps S122 to S125.

Thereafter, the control unit 11 generates a DRR image projected in a projection direction according to the projection condition specified in step S126 based on the CT image of the region of interest (here, a bone region such as the proximal femur) (S17). That is, the control unit 11 generates a DRR image of the region of interest as viewed from the same direction as the capturing direction of the region of interest in the X-ray image. Then, the control unit 11 extracts a half-section image including the left proximal femur from the frontal hip joint X-ray image acquired in step S11 (S18), and stores, as training data, the extracted X-ray image (half-section image of the frontal hip joint X-ray image) and the DRR image of the region of interest generated in step S17 in association with each other in the training DB 12b (S19). In this way, training data, in which the X-ray image of the target site (bone region which is the region of interest) is associated with the DRR image of the target site generated from the CT image highly accurately aligned with the target site in the X-ray image, is accumulated.

Next, the control unit 11 executes similar processing to that of steps S71 to S73 of FIG. 15 (S127 to S129). Specifically, the control unit 11 deletes bone region data from the CT image based on the musculoskeletal labeled image, and extracts muscle region data (S127). Here, the control unit 11 defines a region of interest for each muscle type, and extracts data (CT image) for each region of interest (muscle region). Further, the control unit 11 generates a DRR image projected in the projection direction according to the projection condition specified in step S126 based on the data (CT image) of the extracted region of interest (muscle region) (S128). Then, the control unit 11 associates the X-ray image acquired in step S11 with the DRR image of the region of interest (muscle region) generated in step S128, and stores the images in the training DB 12b as training data (S129). Thereafter, the control unit 11 proceeds to the processing of step S20.

By the above-mentioned processing, alignment based on the bone region is performed for the capturing target (target site or region of interest) in the X-ray image and the CT image, and training data that can efficiently train the learning models 12M and 12Ma of this embodiment can be generated based on the X-ray image and the CT image after alignment. By executing the training process illustrated in FIG. 6 using the training data generated in this way, the information processing apparatus 10 of this embodiment can generate the learning model 12M that can highly accurately predict the DRR image of the bone region, which is a capturing target of an X-ray image, from the X-ray image, and the learning model 12Ma that can highly accurately predict the DRR image of the muscle region, which is a capturing target of an X-ray image, from the X-ray image. In addition, by using the learning models 12M and 12Ma, the information processing apparatus 10 of this embodiment can generate a DRR image of the bone region predicted with high accuracy from the X-ray image, and a DRR image of the muscle region predicted with high accuracy from the X-ray image, and can estimate the bone density and muscle mass of the target site with high accuracy.

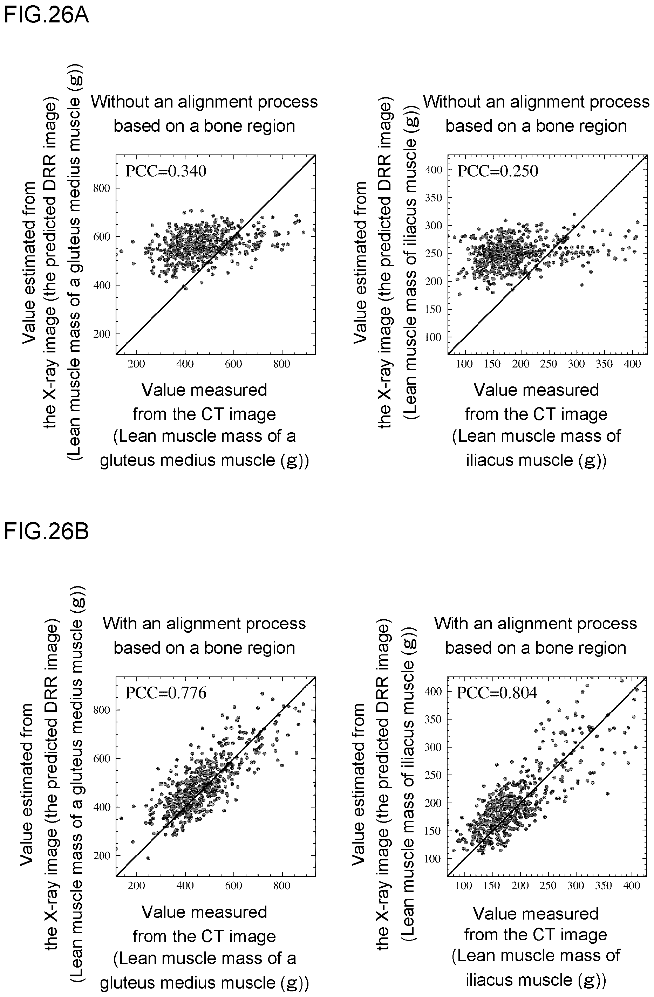

FIGS. 26A and 26B are explanatory views illustrating effects of an alignment process based on the bone region. FIG. 26A is a chart illustrating a relationship between muscle mass (specifically, lean muscle mass) of a muscle region calculated (estimated) from a generated predicted DRR image after generating the predicted DRR image of the muscle region from an X-ray image using the learning model 12Ma trained using training data in which the X-ray image and the DRR image generated from a CT image are associated with each other without performing an alignment process based on a bone region on the X-ray image and the CT image illustrated in this embodiment and muscle mass (specifically, lean muscle mass) of the muscle region measured from the CT image. In the chart of FIG. 26A, a vertical axis represents lean muscle mass estimated from the predicted DRR image, a horizontal axis represents lean muscle mass measured from the CT image, a chart of a gluteus medius muscle is illustrated on a left side of FIG. 26A, and a chart of an iliacus muscle is illustrated on a right side thereof. FIG. 26B is a chart illustrating a relationship between muscle mass (specifically, lean muscle mass) of a muscle region calculated (estimated) from a generated predicted DRR image after generating the predicted DRR image of the muscle region from an X-ray image using the learning model 12Ma trained using training data in which the X-ray image and the DRR image generated from a CT image are associated with each other after performing the alignment process based on a bone region on the X-ray image and the CT image illustrated in this embodiment and muscle mass (specifically, lean muscle mass) of the muscle region measured from the CT image. In the chart of FIG. 26B, a vertical axis represents lean muscle mass estimated from the predicted DRR image, a horizontal axis represents lean muscle mass measured from the CT image, a chart of a gluteus medius muscle is illustrated on a left side of FIG. 26B, and a chart of an iliacus muscle is illustrated on a right side thereof. For example, each of the charts illustrated in FIG. 26A and FIG. 26B illustrates a comparison result (verification result) of lean muscle mass estimated from predicted DRR images predicted from X-ray images collected from 525 patients using the learning model 12Ma trained using 390 pairs of training data generated from X-ray images and CT images collected from patients each suffering from osteoarthritis of the hip joint and lean muscle mass measured from a CT image of each patient.

As can be seen from the charts on the left side of FIG. 26A and on the left side of FIG. 26B, a correlation coefficient (PCC: Pearson Correlation Coefficient) between the estimated value from the predicted DRR image and the measured value from the CT image when alignment of this embodiment is not performed on the lean muscle mass of the gluteus medius muscle is 0.340, the correlation coefficient when alignment of this embodiment is performed is 0.776, and a higher correlation value is obtained when alignment of this embodiment is performed. Similarly, as can be seen from the charts on the right side of FIG. 26A and on the right side of FIG. 26B, the correlation coefficient when alignment of this embodiment is not performed on the lean muscle mass of the iliacus muscle is 0.250, the correlation coefficient when alignment of this embodiment is performed is 0.804, and a higher correlation value is obtained when alignment of this embodiment is performed. Therefore, by training the learning model 12Ma using training data generated by an X-ray image and a CT image after the capturing target is aligned by the alignment process of this embodiment, even when the learning model 12Ma is trained using a small amount of training data, it is possible to realize the learning model 12Ma capable of predicting a DRR image allowing muscle mass to be estimated to the same extent as a measurement value from the CT image. Therefore, workload in a collection process and a training process for the training data can be reduced.

The charts of FIG. 26A and FIG. 26B indicate that more accurate lean muscle mass is obtained when alignment of this embodiment is performed for lean muscle mass of gluteus medius muscle and iliacus muscle. However, a verification process by the present inventor produces similar results for muscle volumes of the gluteus medius muscle and the iliacus muscle. Note that the lean muscle mass is muscle mass calculated by calculating a ratio of muscle to fat in each pixel based on a CT value (pixel value) in each muscle region (individual muscle region) in a CT image, calculating muscle mass of each pixel based on the calculated ratio, and summing masses of the respective pixels. Muscle volume indicates volume of each muscle region (individual muscle region) in the CT image.

In this embodiment, by performing the alignment process on the X-ray image and the CT image based on the bone region, the X-ray image and the CT image after the alignment process are accurately aligned with respect to the muscle region. By training the learning model 12Ma using training data based on the muscle region in the X-ray image aligned in this way and the DRR image of the muscle region generated from the CT image, it is possible to realize the learning model 12Ma that can predict the DRR image of the muscle region capable of estimating muscle mass to the same extent as the muscle mass (for example, lean muscle mass and muscle volume) of each muscle region measured from the CT image. In addition, in this embodiment, by deleting data of the bone region from the aligned CT image, a CT image (data of the muscle region) from which an influence of the bone region is eliminated is extracted, and by using such a CT image, training data used to train the learning model 12Ma can be generated with high accuracy. Therefore, in this embodiment, not only can bone density be estimated with high accuracy by the predicted DRR image of the bone region generated from the X-ray image using the learning model 12M, but muscle mass can also be estimated with high accuracy by the predicted DRR image of the muscle region generated from the X-ray image using the learning model 12Ma.

In this embodiment, when generating training data used to train the learning models 12M and 12Ma, processes are the same as those of each of the above-mentioned embodiments except for a process of aligning the capturing target in the X-ray image and the CT image based on the bone region, and similar effects as those of each of the above-mentioned embodiments can be obtained. In addition, in this embodiment, not only the training data used to train the learning model 12M but also the training data used to train the learning model 12Ma are generated based on the X-ray image and the CT image aligned based on the bone region, so that it is possible to realize the learning model 12Ma that predicts the DRR image of the muscle region in the X-ray image with high accuracy from the X-ray image. In this embodiment, it is possible to apply the modified examples described as appropriate in each of the above-mentioned embodiments.

### (Embodiment 8)

A description will be given of a modified example of the learning model 12Ma that generates a predicted DRR image of a muscle region (for example, gluteus maximus muscle, gluteus medius muscle, iliacus muscle, etc.) included in a target side (for example, lower abdomen, buttocks, etc.) from an X-ray image of the target site in the above-mentioned Embodiments 3 and 7. An information processing apparatus of this embodiment has a similar configuration to that of the information processing apparatus 10 of each of Embodiments 1, 3, and 7, and thus a description of the configuration will be omitted.

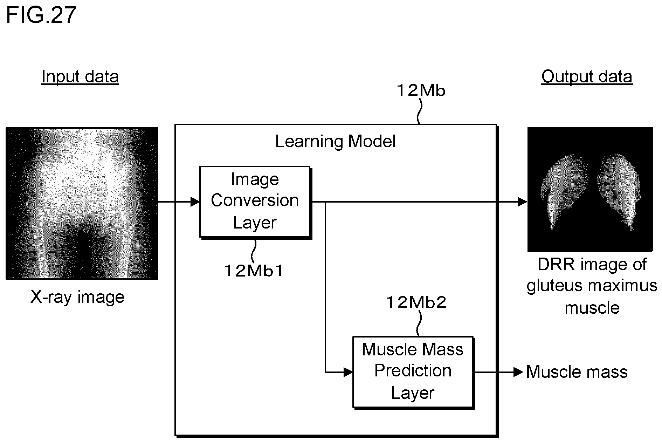

FIG. 27 is an explanatory view illustrating a configuration example of a learning model 12Mb of Embodiment 8. The learning model 12Mb is a model trained to receive input of an X-ray image of a target site, perform calculation to predict a DRR image and muscle mass of a muscle region included in the target site based on the input X-ray image, and output calculation results. The learning model 12Mb illustrated in FIG. 27 is configured to receive input of a frontal hip joint X-ray image and predict a DRR image and muscle mass of a gluteus maximus muscle. Note that the learning model 12Mb is not limited to this configuration, and may be configured, for example, to receive input of a frontal hip joint X-ray image and predict a DRR image and muscle mass of another muscle region such as a gluteus medius muscle or an iliacus muscle, or configured to predict a DRR image and muscle mass for each of plurality of muscle regions. In addition, the learning model 12Mb may be configured to receive input of a frontal chest X-ray image and predict a DRR image and muscle mass of a muscle region such as a pectoralis major, pectoralis minor, subclavius, serratus anterior, intercostal muscle, trapezius, latissimus dorsi, teres major, or erector spinae. The muscle mass may be information indicating some quantity related to muscle, such as lean muscle mass, muscle volume, or muscle density.

The learning model 12Mb of this embodiment has an image conversion layer 12Mb1 and a muscle mass prediction layer 12Mb2. An X-ray image, which is input data of the learning model 12Mb, is input to the image conversion layer 12Mb1, and the image conversion layer 12Mb1 generates and outputs a DRR image of a muscle region (for example, gluteus maximus muscle) included in the X-ray image based on the input X-ray image. The DRR image of the muscle region generated by the image conversion layer 12Mb1 is input to the muscle mass prediction layer 12Mb2, and the muscle mass prediction layer 12Mb2 predicts and outputs muscle mass (for example, lean muscle mass, muscle volume, muscle density, etc.) of the muscle region based on the input DRR image of the muscle region.

The image conversion layer 12Mb1 may be configured, for example, as a GAN such as pix2pix, CycleGAN, or StarGAN, a neural network such as VAE or CNN (for example, U-net), or a model based on another learning algorithm, or may be configured by combining a plurality of learning algorithms. Note that, when the image conversion layer 12Mb1 is configured as pix2pix, the image conversion layer 12Mb1 may have a configuration similar to that of the learning model 12Ma illustrated in FIG. 14. The muscle mass prediction layer 12Mb2 can be configured using an algorithm such as CNN, random forest, SVM (Support Vector Machine), or Transformer, and may be configured by combining a plurality of algorithms. Note that the learning model 12Mb does not need to have the muscle mass prediction layer 12Mb2, and instead of the muscle mass prediction layer 12Mb2, the control unit 11 of the information processing apparatus 10 may configured to perform a process of calculating muscle mass of a muscle region based on the sum of pixel values (luminance values) of each pixel in a DRR image of the muscle region generated by the image conversion layer 12Mb1.

The learning model 12Mb of this embodiment is generated by being trained using training data in which a training X-ray image (frontal hip joint X-ray image), a DRR image of a gluteus maximus muscle which is a ground truth, and a muscle mass of the gluteus maximus muscle are associated with each other. For example, the training X-ray image, the ground truth DRR image, and the muscle mass are preferably a frontal hip joint X-ray image, a DRR image, and muscle mass of a subject diagnosed by the DXA device as any one of normal bone density, osteoporosis, and osteopenia. Note that the DRR image of the gluteus maximus muscle which is the ground truth may be a DRR image of the gluteus maximus muscle (muscle region) generated from a CT image obtained by capturing the buttocks (lower abdomen) using the X-ray CT device. In addition, the muscle mass of the gluteus maximus muscle which is the ground truth may be muscle mass of the gluteus maximus muscle calculated by the sum of muscle masses of respective pixels calculated based on a ratio of muscle to fat in each pixel from the CT value (luminance value) in the muscle region (gluteus maximus muscle) in the CT image, or muscle mass of the gluteus maximus muscle measured by the DXA device.

The learning model 12Mb of this embodiment is trained to output the ground truth DRR image and muscle mass included in training data when an X-ray image included in the training data is input. In the training process, the learning model 12Mb performs calculation based on the input X-ray image, predicts a DRR image and muscle mass of the gluteus maximus muscle in the input X-ray image, and outputs a prediction result. Then, the learning model 12Mb compares the predicted DRR image with the ground truth DRR image, compares the predicted muscle mass with the muscle mass which is the ground truth, and optimizes parameters such as weights (coupling coefficients) between nodes in the image conversion layer 12Mb1 and the muscle mass prediction layer 12Mb2 using the steepest descent method, the backpropagation method, etc. so that the values are close to each other. That is, in the process of training the learning model 12Mb in this embodiment, a difference (error, loss related to muscle mass) between the muscle mass which is the ground truth and the muscle mass based on the DRR image of the muscle region generated by the learning model 12Mb can be fed back. Therefore, according to the training process of this embodiment, the learning model 12Mb is generated to predict a DRR image and muscle mass of a muscle region included in an X-ray image with high accuracy when the X-ray image is input.

Note that the learning model 12Mb of this embodiment may be configured to be unable to include the muscle mass prediction layer 12Mb2. In this case, in the process of training the learning model 12Mb, the control unit 11 of the information processing apparatus 10 inputs an X-ray image to the learning model 12Mb to obtain a DRR image of the muscle region from the learning model 12Mb, and calculates muscle mass of the muscle region from the obtained DRR image. Then, the control unit 11 may calculate a difference between the calculated muscle mass and the muscle mass which is the ground truth, and train the learning model 12Mb (image conversion layer 12Mb1) so that the calculated difference becomes small (so that the calculated muscle mass approximates the muscle mass which is the ground truth). Even in such a training process, it is possible to feed back the difference (error, loss related to muscle mass) between the muscle mass based on the DRR image of the muscle region generated by the learning model 12Mb and the muscle mass which is the ground truth, and to generate the learning model 12Mb that can predict the DRR image and muscle mass of the muscle region included in the input X-ray image with high accuracy.

The information processing apparatus 10 of this embodiment can execute the processing illustrated in FIG. 25, generates training data for training the learning model 12Mb by the processing illustrated in FIG. 25, and executes the process of training the learning model 12Mb by the processing illustrated in FIG. 6. Note that, in the processing illustrated in FIG. 25, after the processing of step S128, the control unit 11 of the information processing apparatus 10 calculates the muscle mass of the muscle region based on the DRR image of the muscle region generated in step S128. Then, in step S129, the control unit 11 stores the X-ray image acquired in step S11, the DRR image of the region of interest (muscle region) generated in step S128, and the muscle mass calculated from the DRR image in the training DB 12b in association with each other as training data. Note that the muscle mass of the muscle region may be muscle mass measured by the DXA device, and in this case, for example, the control unit 11 can acquire the muscle mass measured by the DXA device together with the X-ray image and the CT image in step S11, and use the acquired muscle mass as training data. In addition, in the processing illustrated in FIG. 6, the control unit 11 reads one set of an X-ray image, a DRR image and muscle mass stored in the storage unit 12 in step S31, and performs a process of training the learning model 12Mb using the read training data in step S32. Furthermore, the information processing apparatus 10 of this embodiment can perform the processing illustrated in FIG. 16, use the learning model 12Mb generated as described above to predict a DRR image of a muscle region in the X-ray image with high accuracy, and estimate the muscle mass of the muscle region with high accuracy using the predicted DRR image.

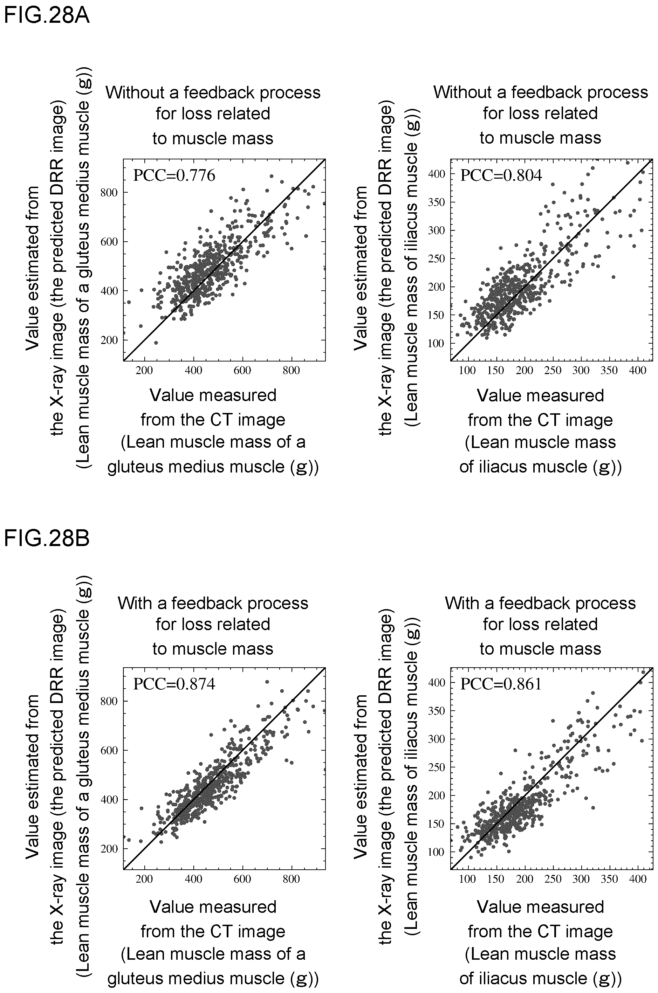

FIGS. 28A and 28B are explanatory views illustrating effects of a feedback process for loss related to muscle mass. FIG. 28A is a chart illustrating a relationship between muscle mass (specifically, lean muscle mass) of a muscle region calculated (estimated) from a generated predicted DRR image after generating the predicted DRR image of the muscle region from an X-ray image using a learning mode (for example, the learning model 12Ma) trained without performing a feedback process for loss related to muscle mass as described above and muscle mass (specifically, lean muscle mass) of the muscle region measured from the CT image. In the chart of FIG. 28A, a vertical axis represents lean muscle mass estimated from the predicted DRR image, a horizontal axis represents lean muscle mass measured from the CT image, a chart of a gluteus medius muscle is illustrated on a left side of FIG. 28A, and a chart of an iliacus muscle is illustrated on a right side thereof. FIG. 28B is a chart illustrating a relationship between muscle mass (specifically, lean muscle mass) of a muscle region calculated (estimated) from a generated predicted DRR image after generating the predicted DRR image of the muscle region from an X-ray image using the learning model 12Mb trained by performing a feedback process for loss related to muscle mass as described above and muscle mass (specifically, lean muscle mass) of the muscle region measured from the CT image. In the chart of FIG. 28B, a vertical axis represents lean muscle mass estimated from the predicted DRR image, a horizontal axis represents lean muscle mass measured from the CT image, a chart of a gluteus medius muscle is illustrated on a left side of FIG. 28B, and a chart of an iliacus muscle is illustrated on a right side thereof. For example, each of the charts illustrated in FIG. 28A and FIG. 28B illustrates a comparison result (verification result) of lean muscle mass estimated from predicted DRR images predicted from X-ray images collected from 525 patients using the learning model 12Mb trained using 390 pairs of training data generated from X-ray images and CT images collected from patients each suffering from osteoarthritis of the hip joint and lean muscle mass measured from a CT image of each patient.

As can be seen from the charts on the left side of FIG. 28A and on the left side of FIG. 28B, a correlation coefficient between the estimated value from the predicted DRR image and the measured value from the CT image when the above-mentioned feedback process for loss related to muscle mass is not performed on the lean muscle mass of the gluteus medius muscle is 0.776, the correlation coefficient when the feedback process is performed is 0.874, and a higher correlation value is obtained when the feedback process for loss related to muscle mass is performed. Similarly, as can be seen from the charts on the right side of FIG. 28A and on the right side of FIG. 28B, the correlation coefficient when the feedback process for loss related to muscle mass is not performed on the lean muscle mass of the iliacus muscle is 0.804, the correlation coefficient when the feedback process is performed is 0.861, and a higher correlation value is obtained when the feedback process for loss related to muscle mass is performed. Therefore, by performing the feedback process for loss related to muscle mass during a process of training the learning model 12Mb, even when the learning model 12Mb is trained using a small amount of training data, it is possible to realize the learning model 12Mb capable of predicting a DRR image allowing muscle mass to be estimated to the same extent as a measurement value from the CT image.

In this embodiment, the charts of FIGS. 28A and 28B indicate that more accurate lean muscle mass is obtained when performing the feedback process for loss related to muscle mass during the process of training the learning model 12Mb for the lean muscle masses of the gluteus medius muscle and the iliacus muscle. However, in a verification process performed by the present inventor, similar results are obtained for muscle volumes of the gluteus medius muscle and the iliacus muscle.

This embodiment is similar to each of the above-mentioned embodiments except a process of feeding back an error (loss) of the muscle mass which is the ground truth with respect to the muscle mass of the muscle region calculated from the DRR image of the muscle region generated by the learning model 12Mb (image conversion layer 12Mb 1) when training the learning model 12Mb, and obtains similar effects to those of each of the above-mentioned embodiments. In addition, in this embodiment, in the process of training the learning model 12Mb, in addition to the error between the DRR image generated by the image conversion layer 12Mb 1 and the ground truth DRR image, the error (loss) of the muscle mass can be fed back, so that it is possible to realize the learning model 12Mb that can predict the DRR image of the muscle region in the X-ray image with higher accuracy from the X-ray image. In this embodiment, it is possible to apply the modified examples described as appropriate in each of the above-mentioned embodiments.

Each of the above-mentioned embodiments is configured so that a simple X-ray image obtained by capturing a target site using the X-ray device is used as input data for the learning models 12M, 12Ma, and 12Mb. However, the present disclosure is not limited to this configuration. For example, an X-ray image obtained by a DXA device may be used as input data for the learning models 12M, 12Ma, and 12Mb. Even in such a configuration, it is possible to execute similar processing to that when a normal X-ray image (X-ray image captured by the X-ray device) is used as input data, and a similar effect can be obtained. Further, in each of the above-mentioned embodiments, instead of the ground truth DRR image (information on the amount of body tissue at the target site obtained from a CT image) used for training data of the learning models 12M, 12Ma, and 12Mb, a two-dimensional image (information on the amount of body tissue) obtained from a three-dimensional image allowing estimation of a composition of muscles and bones at the target site, such as an MRI (Magnetic Resonance Imaging) image, may be used as training data. Even in such a configuration, it is possible to execute similar processing to that when using the DRR image obtained from the CT image, and similar effects can be obtained.

It is to be noted that the disclosed embodiment is illustrative and not restrictive in all aspects. The scope of the present invention is defined by the appended claims rather than by the description preceding them, and all changes that fall within metes and bounds of the claims, or equivalence of such metes and bounds thereof are therefore intended to be embraced by the claims.

### [Description of Reference Numerals]

- 10: information processing apparatus
- 11: control unit
- 12: storage unit
- 13: communication unit
- 14: input unit
- 15: display unit
- 12a: medical image DB
- 12b: training DB
- 12M: learning model
- 12Ma: learning model
- 12M1: bone density estimation learning model

## Claims

1. A program causing a computer to execute processes of:
acquiring training data including an X-ray image of a target site and information related to an amount of body tissue obtained from a CT (Computed Tomography) image of the target site; and
generating a learning model configured to output information related to an amount of body tissue of a target site in an X-ray image when the X-ray image is input using acquired training data.

2. The program according to claim 1, wherein the program causes the computer to execute a process of generating the learning model configured to output an image representing an amount of the body tissue of the target site when the X-ray image is input.

3. The program according to claim 1 or 2, wherein the program causes the computer to execute processes of:
aligning a position of the target site based on the CT image and a position of the target site based on the X-ray image; and
acquiring the training data including information related to an amount of body tissue of the target site obtained from the CT image after alignment.

4. The program according to any one of claims 1 to 3, wherein the program causes the computer to execute processes of:
classifying the target site in the CT image into a plurality of regions including a bone region and a muscle region based on the CT image;
acquiring the training data including information related to bone density of the classified bone region in the CT image; and
generating the learning model configured to output information related to bone density of the bone region in an X-ray image when the X-ray image is input using the training data.

5. The program according to any one of claims 1 to 4, wherein the program causes the computer to execute processes of:
classifying the target site in the CT image into a plurality of regions including a bone region and a muscle region based on the CT image;
acquiring the training data including information related to muscle mass of the classified muscle region in the CT image; and
generating the learning model configured to output information related to muscle mass of the muscle region in an X-ray image when the X-ray image is input using the training data.

6. The program according to any one of claims 1 to 5, wherein:
the learning model includes a generator configured to generate information related to an amount of body tissue of the target site from an input X-ray image, and a discriminator configured to identify authenticity of information related to an amount of body tissue generated by the generator, and
the program causes the computer to execute a process of generating the learning model by training the generator and the discriminator in an adversarial manner.

7. The program according to any one of claims 1 to 6, wherein the program causes the computer to execute processes of:
acquiring training data including information related to an amount of body tissue of the target site obtained from a CT image of the target site, and information related to an amount of body tissue of a site different from the target site; and
generating a second learning model configured to output information related to an amount of body tissue of a site different from the target site when information related to an amount of body tissue of the target site is input using acquired training data.

8. The program according to any one of claims 1 to 7, wherein the program causes the computer to execute processes of:
specifying a projection condition that maximizes a correlation value between an image obtained by projecting a bone region included in the target site in the CT image and a bone region included in the target site in the X-ray image; and
acquiring the training data including information related to an amount of body tissue of the target site obtained from a projection image obtained by projecting the target site in the CT image under a specified projection condition.

9. The program according to claim 8, wherein the program causes the computer to execute processes of:
deleting data of a bone region from a projection image obtained by projecting the target site in the CT image under the specified projection condition;
acquiring the training data including information related to muscle mass of a muscle region in the projection image from which data of the bone region has been deleted; and
generating the learning model configured to output information related to muscle mass of a muscle region in an X-ray image when the X-ray image is input using the training data.

10. The program according to any one of claims 1 to 9, wherein:
the training data includes an X-ray image of the target site, an image representing a muscle region of the target site obtained from a CT image of the target site, and muscle mass of the muscle region,
the learning model is configured to output an image indicating a muscle region in an X-ray image when the X-ray image is input, and
the learning model is trained so that muscle mass calculated based on an image indicating a muscle region in an X-ray image output by the learning model when the X-ray image included in the training data is input is approximated to muscle mass included in the training data.

11. A program causing a computer to execute processes of:
acquiring an X-ray image of a target site; and
inputting the acquired X-ray image to a learning model trained using training data including an X-ray image of a target site and information related to an amount of body tissue obtained from a CT image of the target site and configured to output information related to an amount of body tissue of a target site in an X-ray image when the X-ray image is input, thereby outputting information related to an amount of body tissue of the target site.

12. The program according to claim 11, wherein the output information related to the amount of body tissue is an image representing an amount of body tissue of the target site.

13. The program according to claim 11 or 12, wherein the output information related to the amount of body tissue is bone density of the target site.

14. The program according to any one of claims 11 to 13, wherein the program causes the computer to execute a process of further outputting information related to an amount of body tissue of a site different from a target site in the X-ray image.

15. The program according to any one of claims 11 to 14, wherein the output information related to the amount of body tissue is muscle mass of the target site.

16. The program according to any one of claims 11 to 15, wherein the learning model is trained to output information related to muscle mass of a muscle region in an X-ray image when the X-ray image is input using the training data including information related to muscle mass of a muscle region in a projection image, obtained by projecting the target site in the CT image, from which data of a bone region is deleted under a projection condition maximizing a correlation value between an image obtained by projecting a bone region included in the target site in the CT image and a bone region included in the target site in the X-ray image.

17. The program according to any one of claims 11 to 16, wherein the learning model is trained using the training data including an X-ray image of the target site, an image representing a muscle region of the target site obtained from a CT image of the target site, and muscle mass of the muscle region so that muscle mass calculated based on an image indicating a muscle region in an X-ray image included in the training data output when the X-ray image is input approximates muscle mass included in the training data.

18. An information processing method in which a computer executes processes of:
acquiring an X-ray image of a target site; and
inputting the acquired X-ray image to a learning model trained using training data including an X-ray image of a target site and information related to an amount of body tissue obtained from a CT image of the target site and configured to output information related to an amount of body tissue of a target site in an X-ray image when the X-ray image is input, thereby outputting information related to an amount of body tissue of the target site.

19. An information processing apparatus comprising a control unit, wherein the control unit is configured to:
acquire an X-ray image of a target site; and
input the acquired X-ray image to a learning model trained using training data including an X-ray image of a target site and information related to an amount of body tissue obtained from a CT image of the target site and configured to output information related to an amount of body tissue of a target site in an X-ray image when the X-ray image is input, thereby outputting information related to an amount of body tissue of the target site.
